# FASCICULE DE BREVET EUROPEEN

(11) **EP 1 363 968 B1**
(45) Date de publication et mention de la délivrance du brevet: **28.06.2006**
(21) Numéro de dépôt: 02700320.1
(22) Date de dépôt: 14.01.2002
(51) Int. Cl.: C08J 9/00, C08G 81/02, C08L 101/14, A61K 8/04, A61K 8/60, A61K 8/91, A61Q 1/14, A61Q 5/02, A61Q 19/10

(54) **COMPOSITION MOUSSANTE THERMOGELIFIANTE ET MOUSSE OBTENUE**
THERMOGELIERENDE SCHAUMZUSAMMENSETZUNG UND DARAUS HERGESTELLTER SCHAUMSTOFF
HEAT-GELLING FOAMING COMPOSITION AND RESULTING FOAM

(30) Priorité: 15.01.2001 FR 0100485
(43) Date de publication de la demande: 26.11.2003
(73) Titulaire: L'OREAL S.A., 75008 Paris Cedex (FR)
(72) Inventeur: L'ALLORET, Florence, F-75013 PARIS (FR)
(74) Mandataire: Poulin, Gérard
(86) Numéro de dépôt international: PCT/FR2002/000123
(87) Numéro de publication internationale: WO 2002/055608

(56) Documents cités:
- WO-A-00/35961
- WO-A-97/05185
- WO-A-98/48768
- WO-A-98/50005
- WO-A-98/51694

## Description

La présente invention a trait à des compositions moussantes thermogélifiantes, c'est-à-dire dont la viscosité devient plus importante lorsque la température augmente. L'invention a également trait à la mousse obtenue à partir de ces compositions moussantes et à l'utilisation de polymères spécifiques dans ces compositions moussantes.

Les compositions et mousses selon l'invention sont essentiellement des compositions pour une application topique et notamment cosmétique ou dermatologique.

Le domaine technique de l'invention peut être défini de manière générale comme celui des mousses. Les mousses sont généralement des dispersions de bulles d'un gaz, tel que de l'air, dans une phase continue, généralement aqueuse.

Les compositions moussantes, formules ou produits moussants utilisés, par exemple dans le domaine cosmétique, contiennent des tensioactifs moussants dont la concentration massique est de 5 à 20 %. Or, les tensioactifs utilisés dans les produits moussants, qui sont des espèces amphiphiles de faible masse molaire, à savoir inférieure à 2 000 g/mole, présentent le désavantage d'avoir un caractère relativement agressif vis-à-vis de la peau. En outre, ces produits ou compositions ou formules, qui sont, à température ambiante, généralement sous forme fluide ou gélifiée, présentent de nombreuses limites quant à leur utilisation. En effet :
- les formules fluides sont difficiles à appliquer en raison de leur faible viscosité qui provoquent des coulures lors de leur application ;
- les formules gélifiées sont obtenues à l'aide de gélifiants hydrophiles, tels que les acides polyacryliques réticulés, en particulier les dérivés commercialisés sous le nom de CARBOPOL® , qui limitent le type de galénique obtenue.

En outre, de manière générale, la phase d'initiation de la mousse pour les formules gélifiées est plus lente que pour les produits ne contenant pas de gélifiant et la formule appliquée sur la peau, après dilution avec de l'eau, a une faible viscosité.

En d'autres termes, si l'on souhaite à l'heure actuelle disposer d'une mousse gélifiée sur la peau, on doit obligatoirement faire appel à une composition moussante qui est, elle-même, gélifiée avec tous les inconvénients associés. La variété des textures, que peut revêtir une composition moussante susceptible de donner une mousse gélifiée lors de son application sur la peau, est donc extrêmement limitée.

Il existe donc un besoin pour une composition moussante qui soit peu agressive pour la peau, qui présente des propriétés moussantes importantes, notamment à l'application sur la peau et/ou en présence d'eau chaude, par exemple de 30 à 40°C, qui donne des mousses stables, et, enfin, qui puisse revêtir de nombreuses textures associées à une application aisée.

Le but de la présente invention est, entre autres, de répondre à ce besoin.

Ce but et d'autres encore sont atteints, conformément à l'invention, par une composition moussante thermogélifiante comprenant une phase aqueuse, ladite phase aqueuse comprenant un polymère comprenant des unités hydrosolubles et des unités présentant dans l'eau une température inférieure critique de démixtion LCST, la température de démixtion par chauffage en solution aqueuse desdites unités à LCST étant de 5 à 40°C pour une concentration massique dans l'eau de 1 % desdites unités, et la concentration dudit polymère dans ladite composition étant telle que sa température de gélification soit dans la plage de 5 à 40°C.

De manière surprenante, il a été montré que les compositions moussantes, selon l'invention, qui comprennent le polymère spécifique, tel qu'il est défini ci-dessus, à la concentration spécifique définie ci-dessus, permettent de répondre aux besoins énumérés ci-dessus et de répondre à l'ensemble des exigences mentionnées dans ce qui précède. Notamment le polymère permet d'obtenir une composition qui gélifie à l'application et donne une mousse stable.

En outre, les compositions moussantes, selon l'invention peuvent éventuellement comporter peu ou pas de tensioactifs.

De ce fait, les compositions ou formules moussantes, selon l'invention, présentent une très bonne innocuité vis-à-vis de la peau.

Les compositions moussantes selon l'invention ont des propriétés moussantes importantes et produisent une mousse stable, notamment au-delà de 30°C et éventuellement en présence d'eau chaude.

Les compositions moussantes selon l'invention peuvent se présenter sous toute forme à une température qui est inférieure à leur température de gélification, par exemple, à la température ambiante.

C'est-à-dire qu'à une température inférieure à leur température de gélification, par exemple, à la température ambiante, les compositions de l'invention peuvent avoir une viscosité faible ou bien élevée. Cela signifie que « dans le pot », c'est-à-dire préalablement à une augmentation de température intervenant généralement au moment de leur utilisation, par exemple de leur application, généralement sur la peau, et à la formation de mousse intervenant de même généralement au moment de cette utilisation, la gamme des textures accessibles par les compositions de l'invention n'est pas limitée.

Au contraire, les compositions analogues de l'art antérieur ne peuvent revêtir qu'un nombre extrêmement limité de textures et doivent obligatoirement se présenter sous forme gélifiée, si l'on souhaite obtenir un gel, par exemple, lors de l'application et de la formation concomitante de la mousse.

Selon l'invention, il n'y a aucune restriction sur la forme de la composition à température ambiante.

On peut faire varier à volonté la forme de la composition moussante, et cependant, quelle que soit cette forme, on aura lors de l'application, par exemple sur la peau (dont la température est généralement voisine de 32°C) qui s'accompagne d'une augmentation de température, une apparition du pouvoir gélifiant du polymère et gélification avec formation d'une mousse stable et durable. Cette texture, grâce à la gélification, est d'une application aisée et agréable.

De plus, il a été constaté que les polymères inclus dans la composition moussante de l'invention ne « tuaient pas » la mousse contrairement aux polymères gélifiants de l'art antérieur et que celle-ci était stable sur une longue durée.

En d'autres termes, les compositions moussantes selon l'invention sont, par exemple, sous forme fluide à la température ambiante, et leur viscosité devient importante à une température plus élevée, qui est celle, par exemple, rencontrée lors de l'application sur la peau. Une telle gélification, provoquée à la température de la peau, généralement voisine de 32°C, permet une application aisée des compositions ou formules selon l'invention. Le phénomène de gélification peut également être renforcé en présence d'eau chaude, généralement à une température de 30 à 40°C.

L'invention concerne également la mousse susceptible d'être obtenue à partir de la composition moussante décrite plus haut, cette mousse étant formée d'une dispersion de bulles de gaz dans la phase aqueuse continue. Cette mousse possède toutes les propriétés inhérentes à la mise en oeuvre du polymère spécifique décrit ci-dessus, à savoir, essentiellement le fait que la mousse obtenue est stable au-delà de la température de gélification, par exemple, de la température d'application notamment sur la peau.

L'invention a trait à l'utilisation du polymère, tel que décrit dans la présente description, pour stabiliser une mousse à une température supérieure à sa température de gélification.

L'invention concerne également l'utilisation cosmétique d'une composition selon l'invention, pour le nettoyage et/ou le démaquillage de la peau, du cuir chevelu, des ongles, des cils, des sourcils, des yeux, des muqueuses, des semi-muqueuses et/ou des cheveux.

L'invention est enfin relative à un procédé cosmétique de nettoyage et/ou de démaquillage des matières kératiniques (peau, cuir chevelu, ongles, cils, sourcils, yeux, muqueuses, semi-muqueuses et/ou cheveux), caractérisé par le fait qu'on applique la composition de l'invention, sur les matières kératiniques, en présence d'eau, et qu'on élimine la mousse formée et les résidus de salissure par rinçage à l'eau.

Le constituant essentiel des compositions selon l'invention est un polymère comprenant des unités hydrosolubles et des unités présentant dans l'eau une température inférieure critique de démixtion (LCST), encore appelées « unités à LCST ».

A ce propos, il est utile de rappeler que par unités à LCST, on entend des unités dont la solubilité dans l'eau est modifiée au-delà d'une certaine température. Il s'agit d'unités présentant une température de démixtion par chauffage (ou point de trouble) définissant leur zone de solubilité dans l'eau. La température de démixtion minimale obtenue en fonction de la concentration en polymère constitué uniquement d'unités à LCST est appelée « LCST » (Lower Critical Solution Température). Pour chaque concentration en polymère à LCST, une température de démixtion par chauffage est observée. Elle est supérieure à la LCST qui est le point minimum de la courbe. En dessous de cette température, le polymère est soluble dans l'eau, au-dessus de cette température, le polymère perd sa solubilité dans l'eau.

Ces unités à LCST du polymère utilisé dans la composition moussante selon l'invention répondent à une définition spécifique qui, de manière fondamentale, permet de communiquer au polymère les comprenant les propriétés de thermogélification avantageuses, décrites plus haut.

Ces unités à LCST du polymère ont, selon l'invention, une température de démixtion par chauffage de 5 à 40°C pour une concentration massique dans l'eau de 1 % en poids desdites unités à LCST.

De préférence, la température de démixtion par chauffage en solution aqueuse des unités à LCST du polymère est de 10 à 35°C pour une concentration massique dans l'eau de 1 % desdites unités à LCST.

De préférence, la concentration du polymère est telle que la température de gélification soit dans la plage de 10 à 35°C.

Le polymère ayant la structure décrite plus haut avec des unités hydrosolubles et des unités à LCST spécifiques définies ci-dessus présente en solution aqueuse, des propriétés de gélification au-delà d'une température critique, ou propriétés de thermogélification.

Ces propriétés de gélification par chauffage observées au-delà de la température de démixtion des chaînes à LCST sont décrites dans l'art antérieur, notamment dans les documents [1], [2] et [3]. Elles sont dues à l'association des chaînes à LCST au sein de microdomaines hydrophobes au-delà de leur température de démixtion, formant ainsi des noeuds de réticulation entre les chaînes principales.

Ces propriétés gélifiantes sont observées lorsque la concentration en polymère est suffisante pour permettre des interactions entre des greffons à LCST portées par des macromolécules différentes. La concentration minimale nécessaire, appelée « concentration critique d'agrégation ou CAC », est évaluée par des mesures de rhéologie : il s'agit de la concentration à partir de laquelle la viscosité d'une solution aqueuse des polymères de l'invention devient supérieure à la viscosité d'une solution du polymère équivalent ne comportant pas de chaînes à LCST.

Au-delà de la CAC, les polymères de l'invention présente des propriétés de gélification lorsque la température devient supérieure à une valeur critique appelée « température de gélification ou Tgel ». D'après les données de la littérature, un bon accord existe entre Tgel et la température de démixtion des chaînes à LCST, dans les mêmes conditions de concentrations. La température de gélification d'une solution aqueuse d'un polymère de l'invention est déterminée par des mesures de rhéologie : il s'agit de la température à partir de laquelle la viscosité de la solution d'un polymère de l'invention devient supérieure à la viscosité d'une solution du polymère équivalent ne comportant pas de chaînes à LCST.

Les polymères de l'invention se caractérisent par une température de gélification spécifique généralement de 5 à 40°C, de préférence de 10 à 35°C, pour une concentration massique dans l'eau, par exemple égale à 2 % en poids.

Cette température de gélification spécifique permet à ces polymères de communiquer aux compositions de l'invention toutes les propriétés avantageuses citées plus haut et, en particulier, leur grande variété de formes à température ambiante, et leur effet texture mousse gélifiée lors de l'application.

Des polymères comportant, à l'instar de ceux mis en oeuvre dans les compositions de l'invention, des unités hydrosolubles et des unités à LCST et possédant des propriétés de gélification par chauffage observées au-delà de la température de démixtion des chaînes à LCST sont décrites dans les documents déjà cités plus haut.

Le document [1] est relatif au thermo-épaississement réversible de solutions aqueuses de copolymère comprenant un squelette hydrosoluble d'acide polyacrylique avec des greffons de poly(oxyde d'éthylène) (PEO).

Le document [2] concerne le comportement thermo-épaississant en solution aqueuse de polymères comportant un squelette d'acide 2-acrylamido-2-méthylpropanesulfonique (AMPS) et des chaînes latérales de poly(oxyde éthylène).

De même, le document [3] décrit la thermo-association réversible de copolymères à squelette hydrosoluble polyacrylique ou à base d'AMPS avec des greffons de POE.

Les polymères, tels que ceux mentionnés dans les documents [1], [2] et [3] trouvent, en particulier, leur application dans l'industrie pétrolière.

Ainsi, le document [4] décrit-il des polymères thermoviscosifiants à squelette hydrosoluble comportant des segments, ou portant des chaînes latérales, à LCST qui peuvent être utilisés notamment en tant qu'agents épaississants, constituants de fluides de forage ou autres fluides, et fluides de nettoyage industriel.

Le document [5] décrit des polymères analogues à ceux du document [4] et leur utilisation en tant qu'agent anti-sédimentation de suspensions, éventuellement dans les préparations cosmétiques,

Il est à noter qu'aucun des documents [1] à [5] ne mentionne l'incorporation des polymères dans des compositions moussantes et que, en outre, les polymères décrits ne comprennent pas les unités à LCST, spécifiques selon l'invention.

Le document [6] décrit également les copolymères comprenant un squelette constitué par des unités sensibles au pH, par exemple des unités polyacryliques, et des unités sensibles à la température, greffées sur ce squelette. Ces copolymères présentent des propriétés de gélification en température et ils sont utilisés pour la libération et le relargage contrôlé de principes actifs ou pharmaceutiques et, éventuellement cosmétiques, par application topique.

Les unités sensibles à la température des copolymères de ce document ne sont pas les unités à LCST spécifiques des polymères de l'invention. De plus, les polymères selon le document [6] se caractérisent par l'opacité extrêmement gênante des produits obtenus en température, ce qui n'est pas le cas des polymères mis en oeuvre dans les compositions moussantes de l'invention.

En fait, le polymère de ce document est fondamentalement différent de celui de l'invention car il présente globalement pour l'ensemble du polymère une LCST dans la plage de températures de 20 à 40°C, et non une température de gélification.

Les documents [7] et [8] décrivent des systèmes polymères à gélification réversible, comprenant un composant sensible capable d'agrégation, en réponse à un changement dans un « stimulus » extérieur, et un composant structural. Le stimulus extérieur peut être, par exemple, la température.

Le composant sensible au stimulus extérieur est fondamentalement différent des unités à LCST de la demande. En effet, ces composants sensibles au stimulus extérieur sont en fait constitués par au moins un fragment hydrophile et un fragment hydrophobe. Ainsi, le composant sensible peut être un copolymère bloc, tel qu'un « poloxamer », par exemple un PLURONIC® , qui est un polymère bloc d'oxyde éthylène (soluble) et d'oxyde de propylène (insoluble), un tel copolymère bloc s'agrège au niveau microscopique au-delà d'une température critique ne correspondant pas à une LCST. On peut aussi utiliser comme composant sensible un tensioactif non ionique.

Le document [7] concerne plus particulièrement un réseau polymérique formé d'un squelette hydrosoluble polyacrylique et d'un composant sensible de PLURONIC® , qui est enchevêtré dans ledit squelette, sans liaison covalente, ce réseau a donc une structure particulière qui n'a rien de commun avec le polymère de l'invention. Par contre, dans le document [8], il est question de polymères avec des liaisons covalentes.

Ces polymères ont des propriétés de gélification par chauffage et ils peuvent être utilisés dans le domaine pharmaceutique pour la délivrance de médicaments et dans de nombreux autres domaines y compris le domaine des cosmétiques. En ce qui concerne les applications cosmétiques citées seuls des exemples d'émulsions sont mentionnés. Elles contiennent toutes un tensioactif neutre, anionique, ou cationique, ainsi que le polymère jouant le rôle de gélifiant.

L'utilisation des polymères dans des mousses n'est jamais évoquée, ni les effets de stabilisation et de texture obtenus dans l'invention.

Dans ces formulations, le composant sensible du système polymère a un comportement différent de celui d'unités à LCST, telles que celles du polymère de l'invention, lors du chauffage. Ainsi, lorsqu'on chauffe ledit composant sensible (par exemple, poloxamer), à environ 30-40°C, il présente une température de micellisation, c'est-à-dire une agrégation à l'échelle microscopique, puis lorsqu'on le chauffe davantage, une température de LCST très supérieure. Cette LCST correspond à une agrégation à l'échelle macroscopique entre les macromolécules. Il est expliqué dans WO-A-97 00275 [8] aux pages 16 et 17, que la gélification et la LCST sont observées à des températures qui diffèrent d'environ 70°C, la température de gélification correspondant à la température de micellisation du composant sensible, ce qui montre que ces polymères sont différents de ceux de notre demande. En outre, il n'est pas possible, du fait de la synthèse utilisée dans le document [8], de contrôler parfaitement la structure et les propriétés du polymère final obtenu, comme c'est le cas dans les compositions de l'invention.

On connaît encore par le document [9] des compositions cosmétiques utilisant un système polymère à thermogélification réversible, comprenant l'acide polyacrylique et un poloxamer comme dans les documents [7] et [8]. De nouveau, le système polymère de ces documents est fondamentalement différent de ceux mis en oeuvre dans les compositions de l'invention, si bien que les propriétés avantageuses des compositions de l'invention ne peuvent être obtenues.

WO-A-00 35961 [10] décrit la préparation de polymères ayant des propriétés d'épaississement en température par polymérisation en émulsion et l'utilisation de ces polymères dans des compositions pharmaceutiques et cosmétiques. Ces polymères peuvent être des copolymères ayant des unités hydrosolubles et des unités à LCST à base d'oxyde d'alkylène. Il est prévu d'ajouter des tensioactifs non ioniques aux polymères pour renforcer leurs propriétés thermo-épaississantes.

Il ressort de ce qui précède que la mise en oeuvre dans des compositions moussantes des polymères selon l'invention, présentant des unités à LCST spécifiques et des températures de gélification spécifique leur conférant des propriétés de stabilisation des mousses en température et un effet texture gélifiant à l'application, n'est ni décrite ni suggérée dans les documents de l'art antérieur.

Les polymères utilisés dans l'invention peuvent être des polymères séquencés (ou blocs), ou des polymères greffés, qui comprennent, d'une part, des unités hydrosolubles et, d'autre part, des unités à LCST telles que définies ci-dessus.

On précise que, dans le présent texte, les unités hydrosolubles ou les unités à LCST des polymères mis en oeuvre selon l'invention sont définies comme n'incluant pas les groupes liant entre elles, d'une part, lesdites unités hydrosolubles et, d'autre part, lesdites unités à LCST.

Lesdits groupes de liaison sont issus de la réaction, lors de la préparation du polymère, des sites réactifs portés, d'une part, par les précurseurs desdites unités hydrosolubles et, d'autre part, par les précurseurs desdites unités à LCST.

Les polymères employés dans le cadre de l'invention peuvent donc être des polymères séquences, comprenant par exemple des séquences constituées d'unités hydrosolubles alternées avec des séquences à LCST.

Ces polymères peuvent également se présenter sous la forme de polymères greffés dont le squelette est formé d'unités hydrosolubles, ledit squelette étant porteur de greffons constitués d'unités à LCST.

Lesdits polymères peuvent être partiellement réticulés.

Par unités hydrosolubles, on entend généralement que ces unités sont des unités solubles dans l'eau, à une température de 5 à 80°C, à raison d'au moins 10 g/l, de préférence d'au moins 20 g/l.

Toutefois, on entend également par unités hydrosolubles des unités ne possédant pas obligatoirement la solubilité ci-dessus mentionnée, mais qui en solution aqueuse à 1 % en poids, de 5 à 80°C, permettent l'obtention d'une solution macroscopiquement homogène et transparente, c'est-à-dire ayant une valeur de transmittance maximum de la lumière, quelle que soit la longueur d'onde comprise entre 400 et 800 nm, à travers un échantillon de 1 cm d'épaisseur, d'au moins 85 %, de préférence d'au moins 90 %.

Ces unités hydrosolubles ne présentent pas de température de démixtion par chauffage de type LCST.

Ces unités hydrosolubles sont en totalité ou en partie susceptibles d'être obtenues par polymérisation, notamment radicalaire, ou par polycondensation, ou encore sont constituées en totalité ou en partie par des polymères naturels ou naturels modifiés, existants.

A titre d'exemple, les unités hydrosolubles ou sont en totalité ou en partie susceptibles d'être obtenus par polymérisation, notamment radicalaire, d'au moins un monomère choisi parmi les monomères suivants :
- l'acide (méth)acrylique ;
- les monomères vinyliques de formule (I) suivante : 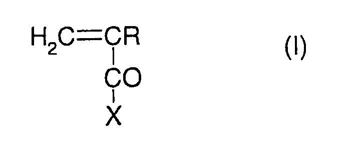
dans laquelle :
- R est choisi parmi H, -CH₃, -C₂H₅ ou -C₃H₇ ; et
- X est choisi parmi :

- les oxydes d'alkyle de type -OR' où R' est un radical hydrocarboné, linéaire ou ramifié, saturé ou insaturé, ayant de 1 à 6 atomes de carbone, éventuellement substitué par au moins un atome d'halogène (iode, brome, chlore, fluor) ; un groupement sulfonique (-SO₃⁻), sulfate (-SO₄⁻), phosphate (-PO₄H₂) ; hydroxy (-OH) ; amine primaire (-NH₂) ; amine secondaire (-NHR₁), tertiaire (-NR₁R₂) ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R' + R₁ + R₂ + R₃ ne dépasse pas 7 ; et
- les groupements -NH₂, -NHR₄ et -NR₄R₅ dans lesquels R₄ et R₅ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₄ + R₅ ne dépasse pas 7, lesdits R₄ et R₅ étant éventuellement substitués par un atome d'halogène (iode, brome, chlore, fluor) ; un groupement hydroxy (-OH) ; sulfonique (-SO₃⁻) ; sulfate (-SO₄⁻) ; phosphate (-PO₄H₂) ; amine primaire (-NH₂) ; amine secondaire (-NHR₁) , tertiaire (-NR₁R₂) et/ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R₄ + R₅ + R₁ + R₂ + R₃ ne dépasse pas 7 ;
- l'anhydride maléique ;
- l'acide itaconique ;
- l'alcool vinylique de formule CH₂=CHOH ;
- l'acétate de vinyle de formule CH₂=CH-OCOCH₃ ;
- les N-vinyllactames tels que la N-vinylpyrrolidone, la N-vinylcaprolactame et la N-butyrolactame ;
- les vinyléthers de formule CH₂=CHOR₆ dans laquelle R₆ est un radical hydrocarboné, linéaire ou ramifié, saturé ou insaturé, ayant de 1 à 6 carbones ;
- les dérivés hydrosolubles du styrène, notamment le styrène sulfonate ;
- le chlorure de diméthyldiallyl ammonium ; et
- la vinylacétamide.

Les polycondensats et les polymères naturels ou naturels modifiés qui peuvent constituer tout ou partie des unités hydrosolubles sont choisis parmi un ou plusieurs des composants suivants :
- les polyuréthanes hydrosolubles,
- la gomme de xanthane, notamment celle commercialisée sous les dénominations Keltrol T et Keltrol SF par Kelco ; ou Rhodigel SM et Rhodigel 200 de Rhodia ;
- les alginates (Kelcosol de Monsanto) et leurs dérivés tels que l'alginate de propylène glycol (Kelcoloid LVF de Kelco) ;
- les dérivés de cellulose et notamment la carboxyméthylcellulose (Aquasorb A500, Hercules), l'hydroxypropylcellulose, l'hydroxyéthylcellulose et l'hydroxyéthylcellulose quaternisée ;
- les galactomannanes et leurs dérivés, tels que la gomme Konjac, la gomme de guar, l'hydroxypropylguar, l'hydroxypropylguar modifié par des groupements méthylcarboxylate de sodium (Jaguar XC97-1, Rhodia), le chlorure de guar hydroxypropyl triméthyl ammonium.

On peut également citer la polyéthylène imine.

De préférence, les unités hydrosolubles ont une masse molaire allant de 1 000 g/mole à 5 000 000 g/mole lorsqu'elles constituent le squelette hydrosoluble d'un polymère greffé.

Ces unités hydrosolubles ont de préférence une masse molaire allant de 500 g/mole à 100 000 g/mole lorsqu'elles constituent un bloc d'un polymère multiblocs.

On peut définir les unités à LCST des polymères utilisés dans l'invention, comme étant des unités dont la solubilité dans l'eau est modifiée au-delà d'une certaine température. Il s'agit d'unités présentant une température de démixtion par chauffage (ou point de trouble) définissant leur zone de solubilité dans l'eau. La température de démixtion minimale obtenue en fonction de la concentration en polymère est appelée "LCST" (Lower Critical Solution Température). Pour chaque concentration en polymère, une température de démixtion par chauffage est observée ; elle est supérieure à la LCST qui est le point minimum de la courbe. En dessous de cette température, le polymère constituant l'unité à LCST est soluble dans l'eau ; au-dessus de cette température, le polymère constituant l'unité à LCST perd sa solubilité dans l'eau.

Certains de ces polymères à LCST sont notamment décrits dans les articles de TAYLOR et al., Journal of Polymer Science, part A : Polymer Chemistry, 1975, 13, 2 551 [11] ; de J. BAILEY et al. , Journal of Applied Polymer Science, 1959, 1,56 [12] ; et de HESKINS et al., Journal of Macromolecular Science, Chemistry A2, 1968, vol. 8, 1 441 [13].

Par soluble dans l'eau à la température T, on entend que les unités présentent une solubilité à T, d'au moins 1 g/l, de préférence d'au moins 2 g/l.

La mesure de la LCST peut se faire visuellement : on détermine la température à laquelle apparaît le point de trouble de la solution aqueuse ; ce point de trouble se traduit par l'opacification de la solution, ou perte de transparence.

D'une manière générale, une composition transparente aura une valeur de transmittance maximum de la lumière, quelle que soit la longueur d'onde comprise entre 400 et 800 nm, à travers un échantillon de 1 cm d'épaisseur, d'au moins 85 %, de préférence d'au moins 90 %.

La transmittance peut être mesurée en plaçant un échantillon de 1 cm d'épaisseur dans le rayon lumineux d'un spectrophotomètre travaillant dans les longueurs d'onde du spectre lumineux.

Les unités à LCST des polymères utilisés dans l'invention peuvent être constituées par un ou plusieurs polymères choisis parmi les polymères suivants :
- les polyéthers tels que le poly oxyde d'éthylène (POE), le poly oxyde de propylène (POP), les copolymères statistiques d'oxyde d'éthylène (OE) et d'oxyde de propylène (OP),
- les polyvinylméthyléthers,
- les dérivés polymériques et copolymériques N-substitués de l'acrylamide ayant une LCST, tels que le poly N-isopropylacrylamide (Nipam) et le poly N-éthylacrylamide, et
- le polyvinylcaprolactame et les copolymères de vinyl caprolacatame.

De préférence, les unités à LCST sont constituées de poly oxyde de propylène (POP)ₙ où n est un nombre entier de 10 à 50, ou de copolymères statistiques d'oxyde d'éthylène (OE) et d'oxyde de propylène (OP), représentés par la formule :

(OE)ₘ (OP)ₙ

dans laquelle m est un nombre entier allant de 1 à 40, de préférence de 2 à 20, et n est un nombre entier allant de 10 à 60, de préférence de 20 à 50.

De préférence, la masse molaire de ces unités à LCST est de 500 à 5 300 g/mole, de préférence encore de 1 500 à 4 000 g/mole.

On a constaté que la répartition statistique des motifs OE et OP se traduit par l'existence d'une température inférieure critique de démixtion, au-delà de laquelle une séparation de phases macroscopique est observée. Ce comportement est différent de celui des copolymères (OE) (OP) à blocs. qui micellisent au-delà d'une température critique dite de micellisation (agrégation à l'échelle microscopique).

Les unités à LCST peuvent donc notamment être des copolymères statistiques d'oxyde d'éthylène et d'oxyde de propylène, aminés, notamment monoaminés, diaminés ou triaminés. Ces polymères, avant réaction, sont porteurs de sites réactifs, dans ce cas de groupes aminés, réagissant avec les sites réactifs des polymères hydrosolubles, par exemple des groupes carboxyle, pour donner le polymère final mis en oeuvre dans l'invention. Dans le polymère final, les unités hydrosolubles sont liés aux unités à LCST par des groupes de liaison issus de la réaction des groupes ou sites réactifs portés respectivement par les unités à LCST et les précurseurs des unités hydrosolubles. Ces groupes de liaison seront, par exemple, des groupes amide, ester, éthers ou uréthanes.

Parmi ces polymères à LCST, commercialement disponibles, on peut citer les copolymères vendus sous la dénomination Jeffamine par HUNTSMAN, et notamment la Jeffamine XTJ-507 (M-2005), la Jeffamine D-2000 et la Jeffamine XTJ-509 (ou T-3000).

Les unités à LCST peuvent également être issues de copolymères OE/OP statistiques à extrémités OH, tels que ceux vendus sous la dénomination Polyglycols P41 et B11 par Clariant.

On peut aussi utiliser dans l'invention comme unités à LCST, les dérivés polymériques et copolymériques N-substitués de l'acrylamide ayant une LCST, ainsi que le polyvinyl caprolactame et les copolymères de vinylcaprolactame.

A titre d'exemples de dérivés polymériques et copolymériques N-substitués de l'acrylamide ayant une LCST, on peut citer le poly N-isopropylacrylamide, le poly N-éthylacrylamide et les copolymères de N-isopropylacrylamide (ou de N-éthylacrylamide) et d'un monomère vinylique choisi parmi les monomères ayant la formule (I) donnée ci-dessus, l'anhydride maléique, l'acide itaconique, la vinylpyrrolidone, le styrène et ses dérivés, le chlorure de diméthyldiallyl ammonium, la vinylacétamide, les vinyléthers et les dérivés de l'acétate de vinyle.

La masse molaire de ces polymères est de préférence de 1 000 g/mole à 500 000 g/mole, de préférence de 2 000 à 50 000 g/mole.

La synthèse de ces polymères peut être réalisée par polymérisation radicalaire à l'aide d'un couple d'amorceurs tel que le chlorhydrate d'aminoéthanethiol, en présence de persulfate de potassium, afin d'obtenir des oligomères précurseurs ayant une extrémité réactive aminée.

A titre d'exemples de copolymères de vinylcaprolactame, on peut citer les copolymères de vinyl caprolactame et d'un monomère vinylique ayant la formule (I) donnée ci-dessus, ou d'un monomère choisi parmi l'anhydride maléique, l'acide itaconique, la vinylpyrrolidone, le styrène et ses dérivés, le chlorure de diméthyldiallyl ammonium, la vinylacétamide, l'alcool vinylique, l'acétate de vinyle, les vinyléthers et les dérivés de l'acétate de vinyle.

La masse molaire de ces polymères ou copolymères de vinylcaprolactame est généralement de 1 000 g/mole à 500 000 g/mole, de préférence de 2 000 à 50 000 g/mol.

La synthèse de ces composés peut être réalisée par polymérisation radicalaire à l'aide d'un couple d'amorceurs tel que le chlorhydrate d'aminoéthanethiol, en présence de persulfate de potassium, afin d'obtenir des unités à LCST ayant une extrémité réactive aminée.

La proportion massique des unités à LCST dans le polymère final est de préférence de 5 % à 70 %, notamment de 20 % à 65 %, et particulièrement de 30 % à 60 % en poids, par rapport au polymère final.

On a vu plus haut que la température de démixtion par chauffage desdites unités à LCST du polymère utilisé dans l'invention est de 5 à 40°C, de préférence de 10 à 35°C, pour une concentration massique dans l'eau, de 1 % en poids desdites unités à LCST.

Les polymères employés dans le cadre de l'invention peuvent être aisément préparés par l'homme du métier sur la base de ses connaissances générales, en utilisant des procédés de greffage, de copolymérisation ou de réaction de couplage.

Lorsque le polymère final se présente sous la forme d'un polymère greffé, notamment présentant un squelette hydrosoluble avec des chaînes latérales ou greffons à LCST, il est possible de le préparer par greffage des unités à LCST ayant au moins une extrémité réactive ou site réactif, notamment aminé(e), sur un polymère hydrosoluble formant le squelette, portant au minimum 10 % (en mole) de groupes réactifs tels que des fonctions acides carboxyliques. Cette réaction peut s'effectuer en présence d'un carbodiimide tel que le dicyclohexylcarbodiimide ou le chlorhydrate de 1-(3-diméthylaminopropyl)-3-éthyl carbodiimide, dans un solvant tel que la N-méthylpyrrolidone ou l'eau.

Une autre possibilité pour préparer des polymères greffés consiste à copolymériser par exemple un macromonomère à LCST (chaîne à LCST précédemment décrite avec une extrémité vinylique) et un monomère vinylique hydrosoluble tel que l'acide acrylique ou les monomères vinyliques ayant la formule (I).

Lorsque le polymère final se présente sous la forme d'un polymère séquencé ou à blocs, il est possible de le préparer par couplage entre des unités hydrosolubles et des unités à LCST, ces unités ayant à chaque extrémité des sites réactifs complémentaires.

Dans le cas des procédés de greffage et des procédés de couplage, les sites réactifs des unités à LCST peuvent être des fonctions amines notamment monoamines, diamines ou triamines, et des fonctions OH. Dans ce cas, les sites réactifs des unités hydrosolubles peuvent être des fonctions acides carboxyliques. Les groupes liant les unités hydrosolubles et les unités à LCST seront donc, par exemple, des groupes amide ou des groupes ester.

Comme on l'a vu précédemment, les compositions moussantes de l'invention comportent essentiellement une phase aqueuse continue.

Selon l'invention, la phase aqueuse comprend un polymère comprenant des unités hydrosolubles et des unités à LCST, tel que défini ci-dessus.

Généralement, la concentration massique en polymère de la phase aqueuse est de 0,1 à 20 %, de préférence de 0,5 à 10 %.

La composition moussante selon l'invention, grâce à la présence du polymère spécifique, décrit plus haut, peut éventuellement ne pas comprendre de tensioactif, c'est-à-dire être exempte de tensioactif
ou en contenir peu, par exemple à une concentration massique égale ou inférieure à 5%, de préférence de 1 à 5 %.

Toutefois, selon l'utilisation finale, la composition de l'invention peut contenir jusqu'à 20 % en poids de tensioactifs, par exemple de 0,05 à 20 % et mieux de 0,1 à 15 % en poids par rapport au poids total de la composition.

Les tensioactifs moussants utilisés peuvent être des tensioactifs non ioniques, anioniques, amphotères ou zwitterioniques ; les tensioactifs non-ioniques étant préférés.

Comme tensioactifs non ioniques, on peut citer par exemple les alkylpolyglycosides (APG), les esters de polyols et d'acides gras, les esters de polyéthylène glycols et d'acide gras, les dérivés d'alcools gras et de polyols (éthers), et les dérivés oxyalkylénés (oxyéthylénés et/ou oxypropylénés) de ces composés. On peut citer aussi les esters de maltose, les alcools gras polyglycérolés, les dérivés de glucamine comme l'éthyl-2 hexyl oxycarbonyl N-méthyl glucamine, et leurs mélanges.

Comme alkylpolyglucosides, on peut citer par exemple le décylglucoside (Alkyl-C9/C11-polyglucoside (1.4)) comme le produit commercialisé sous la dénomination MYDOL 10 par la société Kao Chemicals, le produit commercialisé sous la dénomination PLANTAREN 2000 UP et PLANTACARE 2000 UP par la société Henkel, et le produit commercialisé sous la dénomination ORAMIX NS 10 par la société Seppic ; le caprylyl/capryl glucoside comme le produit commercialisé sous la dénomination ORAMIX CG 110 par la Société Seppic ou sous la dénomination LUTENSOL GD 70 par la Société BASF ; le laurylglucoside comme les produits commercialisés sous les dénominations PLANTAREN 1200 N et PLANTACARE 1200 par la société Henkel ; et le coco-glucoside comme le produit commercialisé sous la dénomination PLANTACARE 818/UP par la société Henkel, et leurs mélanges.

Les dérivés de maltose sont par exemple ceux décrits dans le document EP-A-0 566 438 [14], tels que l'O-octanoyl-6'-D-maltose, ou encore le O-dodécanoyl-6'-D-maltose décrit dans le document FR-A-2 739 556 [15].

Parmi les alcools gras polyglycérolés, on peut citer le dodécanediol polyglycérolé (3,5 moles de glycérol), produit commercialisé sous la dénomination CHIMEXANE NF par la société Chimex.

Comme tensioactifs anioniques, on peut utiliser par exemple les carboxylates, les dérivés des aminoacides, les alkyl sulfates, les alkyl éther sulfates, les sulfonates, les iséthionates, les taurates, les sulfosuccinates, les alkylsulfoacétates, les phosphates et alkylphosphates, les polypeptides, les dérivés anioniques d'alkyl polyglucoside, les savons d'acides gras, et leurs mélanges.

Comme carboxylates, on peut citer par exemple les sels alcalins de N-acylaminoacides ; les amidoéthercarboxylates (AEC) comme le lauryl amidoéther carboxylate de sodium (3 OE) commercialisé sous la dénomination AKYPO FOAM 30 par la société Kao Chemicals ; les sels d'acides carboxyliques polyoxyéthylénés, comme le lauryl éther carboxylate de sodium (C12-14-16 65/25/10) oxyéthyléné (6 OE) commercialisé sous la dénomination AKYPO SOFT 45 NV par la société Kao Chemicals ; les acides gras d'huile d'olive polyoxyéthylénés et de carboxyméthyl, comme le produit commercialisé sous la dénomination OLIVEM 400 par la société Biologia E Tecnologia ; le tri-décyl éther carboxylate de sodium oxyéthyléné (6 OE) commercialisé sous la dénomination NIKKOL ECTD-6NEX par la société Nikkol ; le 2-(2-Hydroxyalkyloxy) acétate de sodium commercialisé sous la dénomination BEAULIGHT SHAA par la société SANYO.

Les dérivés des aminoacides peuvent être choisis par exemple parmi les sarcosinates et notamment les acylsarcosinates comme le lauroylsarcosinate de sodium commercialisé sous la dénomination SARKOSYL NL 97 par la société Ciba ou commercialisé sous la dénomination ORAMIX L 30 par la société Seppic, le myristoyl sarcosinate de sodium, commercialisé sous la dénomination NIKKOL SARCOSINATE MN par la société Nikkol, le palmitoyl sarcosinate de sodium, commercialisé sous la dénomination NIKKOL SARCOSINATE PN par la société Nikkol ; les alaninates comme le N-lauroyl-N-méthylamidopropionate de sodium commercialisé sous la dénomination SODIUM NIKKOL ALANINATE LN 30 par la société Nikkol ou commercialisé sous la dénomination ALANONE ALE par la société Kawaken, et le N-lauroyl N-méthylalanine triéthanolamine, commercialisé sous la dénomination ALANONE ALTA par la société Kawaken ; les N-acylglutamates comme le monococoylglutamate de triéthanolamine commercialisé sous la dénomination ACYLGLUTAMATE CT-12 par la société Ajinomoto, le lauroyl-glutamate de triéthanolamine commercialisé sous la dénomination ACYLGLUTAMATE LT-12 par la société Ajinomoto et le N-lauroyl-L-glutamate monosodique commercialisé sous la dénomination AMISOFT LS-11 par la société Ajinomoto ; les aspartates comme le mélange de N-lauroylaspartate de triéthanolamine et de N-myristoylaspartate de triéthanolamine, commercialisé sous la dénomination ASPARACK LM-TS2 par la société Mitsubishi ; les citrates, et leurs mélanges.

Comme dérivés de la glycine, on peut citer le N-cocoylglycinate de sodium et le N-cocoylglycinate de potassium comme les produits commercialisés sous les dénominations AMILITE GCS-12 et AMILITE GCK-12 par la société Ajinomoto.

Comme alkyl éther sulfates, on peut citer par exemple le lauryl éther sulfate de sodium (C12-14 70/30) (2,2 OE) commercialisé sous les dénominations SIPON AOS 225 ou TEXAPON N702 PATE par la société Henkel, le lauryl éther sulfate d'ammonium (C12-14 70/30) (3 OE) commercialisé sous la dénomination SIPON LEA 370 par la société Henkel, l'alkyl (C12-C14) éther (9 OE) sulfate d'ammonium commercialisé sous la dénomination RHODAPEX AB/20 par la société Rhodia Chimie.

Comme sulfonates, on peut citer par exemple les alpha-oléfines sulfonates comme l'alpha-oléfine sulfonate de sodium (C14-16) commercialisé sous la dénomination BIO-TERGE AS-40 par la société Stepan, commercialisé sous les dénominations WITCONATE AOS PROTEGE et SULFRAMINE AOS PH 12 par la société Witco ou commercialisé sous la dénomination BIO-TERGE AS-40 CG par la société Stepan, l'oléfine sulfonate de sodium secondaire commercialisé sous la dénomination HOSTAPUR SAS 30 par la société Clariant ; les alkyl aryl sulfonates linéaires comme le xylène sulfonate de sodium commercialisé sous les dénominations MANROSOL SXS30, MANROSOL SXS40, MANROSOL SXS93 par la société Manro.

Comme iséthionates, on peut citer les acyliséthionates comme le cocoyl-iséthionate de sodium, tel que le produit commercialisé sous la dénomination JORDAPON CI P par la société Jordan.

Comme taurates, on peut citer le sel de sodium de méthyltaurate d'huile de palmiste commercialisé sous la dénomination HOSTAPON CT PATE par la société Clariant ; les N-acyl N-méthyltaurates comme le N-cocoyl N-méthyltaurate de sodium commercialisé sous la dénomination HOSTAPON LT-SF par la société Clariant ou commercialisé sous la dénomination NIKKOL CMT-30-T par la société Nikkol, le palmitoyl méthyltaurate de sodium commercialisé sous la dénomination NIKKOL PMT par la société Nikkol.

Comme sulfosuccinates, on peut citer par exemple le monosulfosuccinate d'alcool laurylique (C12/C14 70/30) oxyéthyléné (3 OE) commercialisé sous les dénominations SETACIN 103 SPECIAL, REWOPOL SB-FA 30 K 4 par la société Witco, le sel di-sodique d'un hémisulfosuccinate des alcools C12-C14, commercialisé sous la dénomination SETACIN F SPECIAL PASTE par la société Zschimmer Schwarz, l'oléamidosulfosuccinate di-sodique oxyéthyléné (2 OE) commercialisé sous la dénomination STANDAPOL SH 135 par la société Henkel, le monosulfosuccinate d'amide laurique oxyéthyléné (5 OE) commercialisé sous la dénomination LEBON A-5000 par la société Sanyo, le sel di-sodique de monosulfosuccinate de lauryl citrate oxyéthyléné (10 OE) commercialisé sous la dénomination REWOPOL SB CS 50 par la société Witco, le monosulfosuccinate de monoéthanolamide ricinoléique commercialisé sous la dénomination REWODERM S 1333 par la société Witco.

Comme phosphates et alkylphosphates, on peut citer par exemple les monoalkylphosphates et les dialkylphosphates, tels que le monophosphate de laurvle commercialisé sous la dénomination MAP 20 par la société Kao Chemicals, le sel de potassium de l'acide dodécylphosphorique, mélange de mono- et di-ester (diester majoritaire) commercialisé sous la dénomination CRAFOL AP-31 par la société Cognis, le mélange de monoester et de di-ester d'acide octylphosphorique, commercialisé sous la dénomination CRAFOL AP-20 par la société Cognis, le mélange de monoester et de diester d'acide phophorique de 2-butyloctanol éthoxylé (7 moles d'OE), commercialisé sous la dénomination ISOFOL 12 7 EO-PHOSPHATE ESTER par la société Condea, le sel de potassium ou de triéthanolamine de monoalkyl (C12-C13) phosphate commercialisé sous les références ARLATONE MAP 230K-40 et ARLATONE MAP 230T-60 par la société Uniqema, le lauryl phosphate de potassium commercialisé sous la dénomination DERMALCARE MAP XC-99/09 par la société Rhodia Chimie.

Les polypeptides sont obtenus par exemple par condensation d'une chaîne grasse sur les aminoacides de céréale et notamment du blé et de l'avoine. Comme polypeptides, on peut citer par exemple le sel de potassium de la lauroyl protéine de blé hydrolysée, commercialisé sous la dénomination AMINOFOAM W OR par la société Croda, le sel de triéthanolamine de cocoyl protéine de soja hydrolysée, commercialisé sous la dénomination MAY-TEIN SY par la société Maybrook, le sel de sodium des lauroyl aminoacides d'avoine, commercialisé sous la dénomination PROTEOL OAT par la société Seppic, l'hydrolysat de collagène greffé sur l'acide gras de coprah, commercialisé sous la dénomination GELIDERM 3000 par la société Deutsche Gelatine, les protéines de soja acylées par des acides de coprah hydrogénés, commercialisées sous la dénomination PROTEOL VS 22 par la société Seppic.

Les dérivés anioniques d'alkylpolyglucosides peuvent être notamment des citrates, tartrates, sulfosuccinates, carbonates et éthers de glycérol obtenus à partir des alkyl polyglucosides. On peut citer par exemple le sel de sodium d'ester tartrique de cocoylpolyglucoside (1,4), commercialisé sous la dénomination EUCAROL AGE-ET par la société Cesalpinia, le sel di-sodique d'ester sulfosuccinique de cocoylpolyglucoside (1,4), commercialisé sous la dénomination ESSAI 512 MP par la société Seppic, le sel de sodium d'ester citrique de cocoyl polyglucoside (1,4) commercialisé sous la dénomination EUCAROL AGE-EC® par la société Cesalpinia. Un autre dérivé d'holoside anionique peut être le dodécyl-D-galactoside uronate de sodium commercialisé sous la dénomination DODECYL-D-GALACTOSIDE URONATE DE SODIUM par la société SOLIANCE.

Les savons d'acide gras qui peuvent être utilisés comme tensioactifs anioniques sont des acides gras d'origine naturelle ou synthétique, salifiés par une base minérale ou organique. La chaîne grasse peut comporter de 6 à 22 atomes de carbone, de préférence de 8 à 18 atomes de carbone. La base minérale ou organique peut être choisie parmi les métaux alcalins ou alcalino-terreux, les aminoacides et les aminoalcools. Comme sels, on peut utiliser par exemple les sels de sodium, de potassium, de magnésium, de triéthanolamine, de N-méthylglucamine, de lysine et d'arginine. Comme savons, on peut citer par exemple les sels de potassium ou de sodium des acides laurique, myristique, palmitique, stéarique (laurate, myristate, palmitate et stéarate de potassium ou de sodium), et leurs mélanges.

Comme tensioactifs amphotères et zwitterioniques, on peut utiliser par exemple, les bétaïnes, les N-alkylamidobétaïnes et leurs dérivés, les dérivés de la glycine, les sultaïnes, les alkyl polyaminocarboxylates, les alkylamphoacétates et leurs mélanges.

Comme bétaïnes, on peut citer par exemple la cocobétaïne comme le produit commercialisé sous la dénomination DEHYTON AB-30 par la société Henkel, la laurylbétaïne comme le produit commercialisé sous la dénomination GENAGEN KB par la société Clariant, la laurylbétaïne oxyéthylénée (10 OE), comme le produit commercialisé sous la dénomination LAURYLETHER (10 OE) BETAINE par la société Shin Nihon Rica, la stéarylbétaïne oxyéthylénée (10 OE) comme le produit commercialisé sous la dénomination STEARYLETHER (10 OE)BETAINE par la société Shin Nihon Rica.

Parmi les N-alkylamidobétaines et leurs dérivés, on peut citer par exemple la cocamidopropyl bétaine commercialisée sous la dénomination LEBON 2000 HG par la société Sanyo, ou commercialisée sous la dénomination EMPIGEN BB par la société Albright & Wilson, la lauramidopropyl bétaïne commercialisée sous la dénomination REWOTERIC AMB12P par la société Witco.

Comme sultaïnes, on peut citer le cocoylamidopropylhydroxy-sulfobétaine commercialisé sous la dénomination CROSULTAINE C-50 par la société Croda.

Comme alkyl polyaminocarboxylates (APAC), on peut citer le cocoylpolyamino-carboxylate de sodium, commercialisé sous la dénomination AMPHOLAK 7 CX/C, et AMPHOLAK 7 CX par la société Akzo Nobel, le stéarylpolyamidocarboxylate de sodium commercialisé sous la dénomination AMPHOLAK 7 TX/C par la société Akzo Nobel, la carboxyméthyloléyl-polypropylamine de sodium, commercialisé sous la dénomination AMPHOLAK XO7/C par la société Akzo Nobel.

Comme alkylamphoacétates, on peut citer par exemple le N-cocoyl-N-carboxyméthoxyéthyl-N-carboxyméthyl-éthylènediamine N-di-sodique (nom CTFA : disodium cocamphodiacetate) comme le produit commercialisé sous la dénomination MIRANOL C2M CONCENTRE NP par la société Rhodia Chimie, et le N-cocoyl-N-hydroxyéthyl-N-carboxyméthyl-éthylènediamine N-sodique (nom CTFA : sodium cocamphoacétate).

La phase aqueuse peut éventuellement, en outre, comprendre un agent gélifiant à une concentration massique de 0,01 à 20 % du poids total de la composition.

Dans les compositions moussantes de l'invention, la phase aqueuse est de préférence constituée par un milieu physiologiquement acceptable permettant une application topique et notamment cosmétique.

On entend dans la présente demande par "milieu physiologiquement acceptable" un milieu compatible avec toutes les matières kératiniques telles que la peau y compris le cuir chevelu, les ongles, les muqueuses, les yeux et les cheveux ou toute autre zone cutanée du corps.

Le milieu physiologiquement acceptable des compositions moussantes de l'invention comprend de l'eau. La quantité d'eau peut aller de 30 à 99,98 % en poids et de préférence de 40 à 95 % en poids par rapport au poids total de la composition.

L'eau utilisée peut être outre de l'eau, une eau florale telle que l'eau de bleuet, une eau minérale telle que l'eau de Vittel, l'eau de LUCAS ou l'eau de la Roche Posay et/ou une eau thermale.

Le milieu physiologiquement acceptable peut contenir, outre l'eau, un ou plusieurs solvants choisis parmi les alcools inférieurs comportant de 1 à 8 atomes de carbone, tels que l'éthanol ; les polyols tels que la glycérine ; les glycols comme le butylène glycol, l'isoprène glycol, le propylène glycol, les polyéthylène glycols tels que le PEG-8 ; le sorbitol ; les sucres tels que le glucose, le fructose, le maltose, le lactose et le sucrose ; et leurs mélanges. La quantité de solvant(s) peut aller de 0,5 à 30 % en poids et de préférence de 5 à 20 % en poids par rapport au poids total de la composition.

Les compositions moussantes de l'invention peuvent encore contenir des adjuvants habituels dans les domaines cosmétique et dermatologique, tels que les charges minérales ou organiques, les actifs hydrophiles
ou lipophiles, les conservateurs, les gélifiants, les plastifiants, les antioxydants, les parfums, les absorbeurs d'odeur, les filtres UV, les séquestrants (EDTA), les ajusteurs de pH acides ou basiques ou des tampons, et des matières colorantes (pigments ou colorants ou nacres). Les quantités de ces différents additifs sont celles classiquement utilisées dans les domaines considérés, et par exemple de 0,01 à 20 % du poids total de la composition. Bien entendu, l'homme de l'art veillera à choisir le ou les éventuels composés à ajouter aux compositions moussantes selon l'invention de manière telle que les propriétés avantageuses attachées intrinsèquement à ces compositions ne soient pas, ou substantiellement pas, altérées par l'addition envisagée.

Par charges, il faut comprendre des particules incolores ou blanches, minérales ou de synthèse, lamellaires ou non lamellaires, destinées à donner du corps ou de la rigidité à la composition et/ou de la douceur, de la matité et de l'uniformité au maquillage. Comme charges, on peut notamment citer le talc, le mica, la silice, le nitrure de bore, l'oxychlorure de bismuth, le kaolin, les poudres de Nylon telles que Nylon-12 (Orgasol commercialisé par la société Atochem), les poudres de polyéthylène, le Téflon (poudres de polymères de tétrafluoroéthylène), les poudres de polyuréthanne, les poudres de polystyrène, les poudres de polyester, l'amidon éventuellement modifié, des microsphères de copolymères, telles que celles commercialisées sous les dénominations Expancel par la société Nobel Industrie, les microéponges comme le Polytrap commercialisé par la société Dow Corning, les microbilles de résine de silicone telles que celles commercialisées par la société Toshiba sous la dénomination Tospearl, le carbonate de calcium précipité, le carbonate et l'hydrocarbonate de magnésium, l'hydroxyapatite, les microsphères de silice creuses (Silica Beads de la Société Maprecos), les microcapsules de verre ou de céramique, les savons métalliques dérivés d'acides organiques carboxyliques ayant de 8 à 22 atomes de carbone, de préférence de 12 à 18 atomes de carbone, par exemple le stéarate de zinc, de magnésium ou de lithium, le laurate de zinc, le myristate de magnésium, et leurs mélanges.

Par pigments, il faut comprendre des particules blanches ou colorées, minérales ou organiques, insolubles dans le milieu, destinées à colorer et/ou opacifier la composition. Ils peuvent être blancs ou colorés, minéraux et/ou organiques, de taille usuelle ou nanométrique. On peut citer, parmi les pigments et les nanopigments minéraux, les dioxydes de titane, de zirconium ou de cérium, ainsi que les oxydes de zinc, de fer ou de chrome, les nanotitanes (nanopigments de dioxyde de titane), les nanozincs (nanopigments d'oxyde de zinc), le bleu ferrique. Parmi les pigments organiques, on peut citer le noir de carbone, et les laques comme les sels de calcium, de baryum, d'aluminium ou de zirconium, de colorants acides tels que les colorants halogéno-acides, azoïques ou anthraquinoniques.

Par nacres, il faut comprendre des particules irisées qui réfléchissent la lumière. Parmi les nacres envisageables, on peut citer la nacre naturelle, le mica recouvert d'oxyde de titane, d'oxyde de fer, de pigment naturel ou d'oxychlorure de bismuth ainsi que le mica titane coloré.

On peut ajouter aux compositions de l'invention un agent gélifiant afin de régler la texture de la composition et d'accéder à une large gamme de textures allant du lait à la crème. Comme on l'a déjà mentionné plus haut, les compositions selon l'invention peuvent à faible température, par exemple à température ambiante, « dans le pot », revêtir toute texture souhaitable. Il n'y a aucune restriction sur la texture que la composition peut avoir, avant application. En particulier, la composition ne doit pas contenir obligatoirement un agent gélifiant pour que lors de l'application, on obtienne une mousse gélifiée. En effet, grâce au polymère spécifique inclus dans la composition de l'invention, la mousse obtenue lors de l'augmentation de température se produisant, par exemple, lors de l'application de la composition, notamment sur la peau est gélifiée, stable, quelle que soit la texture, la forme de la composition moussante avant application dans le pot. On n'inclura donc un gélifiant dans la composition que si on souhaite que celle-ci ait un aspect gélifié, cet aspect n'étant selon l'invention qu'un aspect particulier parmi la multitude d'aspects, de textures, de formes que peut avoir la composition moussante.

Toutefois, selon un mode préféré de réalisation de l'invention, la composition a pas ou peu d'agent gélifiant (moins de 0,1 %).

Les gélifiants utilisables peuvent être des gélifiants hydrophiles. A titre d'exemples de gélifiants hydrophiles, on peut citer en particulier les polymères carboxyvinyliques (carbomer), les copolymères acryliques tels que les copolymères d'acrylates/alkylacrylates, les polyacrylamides, les polysaccharides, les gommes naturelles et les argiles.

La composition peut contenir éventuellement une phase huileuse.

Cette La phase huileuse comporte de préférence au moins une huile.

Comme huiles utilisables dans la composition de l'invention, on peut citer par exemple :
- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéarylmalate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- des huiles essentielles naturelles ou synthétiques telles que, par exemple, les huiles d'eucalyptus, de lavandin, de lavande, de vétiver, de litsea cubeba, de citron, de santal, de romarin, de camomille, de sarriette, de noix de muscade, de cannelle, d'hysope, de carvi, d'orange, de géraniol, de cade et de bergamote ;
- les alcools gras ayant de 8 à 26 atomes de carbone, comme l'alcool cétylique, l'alcool stéarylique et leur mélange (alcool cétylstéarylique), l'octyl dodécanol, le 2-butyloctanol, le 2-hexyldécanol, le 2-undécylpentadécanol, l'alcool oléique ou l'alcool linoléique ;
- les huiles fluorées partiellement hydrocarbonées et/ou siliconées comme celles décrites dans le document JP-A-2-295912 ;
- les huiles de silicone comme les polyméthylsiloxanes (PDMS) volatiles ou non à chaîne siliconée linéaire ou cyclique, liquides ou pâteux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphénylsiloxanes, les diphényl-diméthicones, les diphénylméthyldiphényl trisiloxanes, les 2-phényléthyltriméthyl-siloxysilicates, et les polyméthylphénylsiloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol) ; les résines de silicone telles que la trifluorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées.

Quand elle est présente, la quantité de phase huileuse peut aller par exemple de 0,01 à 50 % en poids et de préférence de 0,1 à 30 % en poids par rapport au poids total de la composition.

Les compositions moussantes de l'invention peuvent se présenter notamment sous la forme d'une composition cosmétique, notamment de nettoyage ou de démaquillage, susceptible d'être appliquée sur la peau y compris le cuir chevelu, les ongles, les cheveux, les cils, les sourcils, les yeux, les muqueuses et les semi-muqueuses, et toute autre zone cutanée du corps et du visage.

La composition selon l'invention peut constituer un gel douche, un nettoyant pour le visage, un produit démaquillant, un shampooing, une mousse ou un gel à raser, etc..

A partir de la composition moussante selon l'invention, c'est-à-dire d'une composition susceptible de mousser, mais qui ne mousse pas notamment dans le récipient qui la contient, lors du stockage, on obtient une mousse, c'est-à-dire une dispersion de bulles de gaz dans une phase continue aqueuse en exerçant une action mécanique, telle qu'une agitation, sur ladite composition. Cette action mécanique peut être due à l'action des doigts de l'utilisateur lors de l'application ou bien elle peut être due au passage de la composition par une buse ou autre mélangeur statique permettant, par exemple, de mélanger un courant de la composition avec un courant de gaz pour former des bulles de ce gaz dans la phase aqueuse. Le gaz inclus dans la mousse est généralement de l'air, mais il peut s'agir aussi d'azote ou d'autres gaz propulseurs connus dans le telles applications, en particulier de gaz hydrocarbonés comme par exemple le propane, le n-butane, l'isobutane et leurs mélanges.

L'invention a aussi pour objet l'utilisation cosmétique de la composition moussante telle que définie ci-dessus pour le nettoyage et/ou le démaquillage de la peau, du cuir chevelu, des ongles, des cils, des sourcils, des yeux, des muqueuses, des semi-muqueuses et/ou des cheveux.

Les compositions moussantes selon l'invention peuvent par exemple être utilisées de deux façons :
- la première utilisation consiste à étaler le gel dans les mains, à l'appliquer sur le visage ou sur le corps puis à le masser en présence d'eau pour développer la mousse directement sur le visage ou le corps ;
- l'autre utilisation possible de ce type de produit consiste à développer la mousse dans les paumes des mains avant d'être appliquée sur le visage ou le corps.

Puis, la mousse est ensuite rincée. Un autre objet de l'invention consiste en un procédé cosmétique de nettoyage et/ou de démaquillage des matières kératiniques (peau, cuir chevelu, ongles, cils, sourcils, yeux, muqueuses, semi-muqueuses et/ou cheveux), caractérisé par le fait qu'on applique la composition de l'invention, sur les matières kératiniques, en présence d'eau, et qu'on élimine la mousse formée et les résidus de salissure par rinçage à l'eau.

D'autres caractéristiques et avantages de l'invention apparaîtront mieux à la lecture de la description qui suit donnée à titre illustratif et non limitatif, en référence aux dessins joints dans lesquels :
- la figure 1 est un graphique qui donne la viscosité V (en Pa.s), en fonction de la température T (°C) d'une solution aqueuse du polymère 2 du Tableau 1, à 1 % en poids (exemple 2) ;
- la figure 2 est un graphique qui donne la viscosité V (en Pa.s), en fonction de la température T (°C) d'une solution aqueuse contenant le polymère 2 du Tableau 1 à 1 % en poids et un tensioactif (exemple 3).

### Description détaillée des modes de réalisation

Les exemples suivants illustrent la réalisation de compositions moussantes selon l'invention comprenant des polymères comportant des unités hydrosolubles et des unités à LCST spécifiques.

Les polymères utilisés dans ces exemples sont constitués par un squelette d'acide polyacrylique (PAA) portant des chaînes latérales ou greffons constituées par des unités à LCST spécifiques. Ils sont caractérisés par la masse molaire du squelette hydrosoluble (acide polyacrylique), la nature chimique des chaînes à LCST, leur proportion massique dans le polymère et leur masse molaire.

Les caractéristiques des polymères utilisés sont données dans le tableau I.

**Tableau I**

| | Squelette hydrosoluble | Greffons (unités à LCST) | Proportion : unités à LCST dans polymère final (en poids) | Taux de greffage (% en mole) |
|---|---|---|---|---|
| Polymère 1 | Acide polyacrylique; PM=450 000 | (OE)₆(OP)₃₉ statistique Jeffamine M-2005; PM=2 600 | 51 % | 3,9% |
| Polymère 2 | Acide polyacrylique; PM=550 000 | PolyN-isopropylacrylamide (pNIPAM) PM= 10 000 | 49 % | 0,9% |

Ces polymères sont préparés de la façon suivante.

### Préparation du Polymère 1

Dans un réacteur de 500 ml muni d'un réfrigérant, on dissout 3 g d'acide polyacrylique de masse molaire moyenne 450 000 g/mole (Aldrich) dans 220 ml de N-méthyl pyrrolidone sous agitation à 60°C pendant 12 h.

On dissout 4,181 g de copolymère (OE)₆(OP)₃₉ statistique monoaminé, de masse molaire 2600 g/mole ayant un point de trouble, à une concentration de 1% en poids dans l'eau, de 16°C (Jeffamine M-2005 de Huntsman) dans 50 ml de N-méthylpyrrolidone sous agitation, à 20°C, pendant 15 min.. La solution obtenue est ajoutée goutte à goutte au milieu réactionnel contenant l'acide polyacrylique, sous vive agitation à 60°C.

On dissout 2,158 g de dicyclohexylcarbodiimide dans 30 ml de N-méthylpyrrolidone sous agitation à 20°C pendant 15 min.. La solution obtenue est ajoutée goutte à goutte au milieu réactionnel contenant l'acide polyacrylique et le copolymère (OE)₆(OP)₃₉ statistique monoaminé, sous vive agitation à 60°C. On agite le mélange final pendant 12 h à 60°C.

On refroidit le mélange à 20°C puis on le place dans un réfrigérateur à 4°C pendant 24 h. Les cristaux de dicyclohexylurée formés sont éliminés par filtration du milieu réactionnel.

Le polymère est alors neutralisé à l'aide de 19 g de soude à 35 % (4 fois en excès par rapport au nombre de mole d'acide acrylique), ce qui conduit à sa précipitation. Après 12 h au repos, le milieu réactionnel est filtré afin de récupérer le polymère précipité. Celui-ci est séché sous vide à 35°C pendant 24 h.

On récupère 13,55 g de solide qui sont dissous dans 2 1 d'eau désionisée. Cette solution est ultrafiltrée à l'aide d'un système d'ultrafiltration Millipore contenant une membrane dont le seuil de coupure est fixée à 10 000 Daltons. La solution ainsi purifiée est lyophilisée afin de recueillir le polymère sous forme solide.

On obtient 7,05 g d'acide polyacrylique (450 000 g/mole) greffé par 3,9 % (en mole) de copolymère (OE)₆(OP)₃₉ statistique monoaminé.

La proportion massiques des unités à LCST dans le polymère final est de 51 %.

Le polymère ainsi obtenu présente une solubilité dans l'eau, à 20°C, d'au moins 10 g/1.

### Préparation du polymère 2

Le polymère 2 qui comporte des greffons de poly-N-isopropylacrylamide (pNIPAM) est préparé par un procédé en 2 étapes :

### 1) Synthèse d'oligomères pNIPAM portant une extrémité réactive aminée

Dans un ballon tricol de 250 ml muni d'un réfrigérant et d'une arrivée d'azote sont introduits 8 g de N-isopropylacrylamide et 80 ml de diméthylsulfoxyde. Ce mélange est chauffé sous agitation à 29°C à l'aide d'un bain-marie et placé sous un barbotage d'azote. Après 45 min., 0,161 g de chlorhydrate d'aminoéthanethiol préalablement dissous dans 4 ml de diméthylsulfoxyde sont ajoutés au milieu réactionnel. 5 min. après, 0,191 g de persulfate de potassium dissous dans 8 ml de diméthylsulfoxyde sont ajoutés au milieu réactionnel. Ce milieu réactionnel est maintenu sous agitation et atmosphère d'azote pendant 3 h à 29°C.

Les oligomères de poly N-isopropylacrylamide (pNIPAM) synthétisés sont isolés par précipitation du milieu réactionnel dans un mélange d'acétone (40 % en volume) et d'hexane (60 %).

### 2) Greffage des oligomères de pNIPAM sur de l'acide polyacrylique

Dans un ballon tricol de 250 ml muni d'un réfrigérant sont dissous dans 100 ml de 1-méthyl 2-pyrrolydone 3 g d'acide polyacrylique de masse molaire 550 000 g/mole, sous agitation à 60°C pendant 12 h. 3,757 g d'oligomères pNIPAM préalablement dissous dans 25 ml de 1-méthyl 2-pyrrolidone sont introduits goutte à goutte dans le milieu réactionnel sous agitation. 15 min. après, 0,776 g de dicyclohexylcarbodiimide préalablement dissous dans 25 ml de 1-méthyl 2-pyrrolidone sont introduits goutte à goutte dans le milieu réactionnel sous vive agitation. Le milieu réactionnel est maintenu pendant 12 h à 60°C sous agitation.

Le milieu réactionnel est alors refroidi à 20°C puis placé dans un réfrigérateur à 4°C pendant 24 h. Les cristaux de dicyclohexylurée formés sont alors éliminés par filtration. Le polymère est alors neutralisé à l'aide de 19 g de soude à 35 % (4 fois en excès par rapport au nombre de mole d'acide acrylique), ce qui conduit à sa précipitation. Après 12 h au repos, le milieu réactionnel est filtré afin de récupérer le polymère précipité. Celui-ci est séché sous vide à 35°C pendant 24 h.

On récupère 10,2 g de solide qui sont dissous dans 2 litres d'eau désionisée. Cette solution est ultrafiltrée à l'aide d'un système d'ultrafiltration Millipore contenant une membrane dont le seuil de coupure est fixée à 10 000 Daltons. La solution ainsi purifiée est lyophilisée afin de recueillir le polymère sous forme solide.

On obtient 4,8 g d'acide polyacrylique (450 000 g/mole) greffé par 0,9% (en mole) de poly N-isopropylacrylamide.

La proportion massique des unités à LCST dans le polymère final est de 49%.

On détermine les concentrations d'agrégation critiques (CAC) des deux polymères préparés plus haut, par rhéologie, selon la méthode décrite précédemment, à l'aide d'un rhéomètre Haake RS 150, équipé d'une géométrie cône/plan (35 mm, 2°) et d'un bain thermostaté, afin de contrôler la température entre 5 et 80°C. Les mesures ont été effectuées dans le mode écoulement à une vitesse de cisaillement de 10 s⁻¹, en faisant varier la température de 15 à 50°C à une vitesse de 0,5°C/minute.

Pour le polymère 1 dans NaCl 0,2 M ; la concentration d'agrégation critique (CAC) est d'environ 1 % en poids.

Tandis que pour le polymère 2 dans l'eau pure, la concentration d'agrégation critique est d'environ 0,3 % en poids.

### Exemple 1

Cet exemple est relatif à des formules moussantes sans tensioactif contenant le polymère 1.

On étudie les stabilités à 15 et 50°C des mousses obtenues à partir de ces formules ainsi que leur viscosité à ces températures.

### Préparation de la formule moussante

On prépare la formule 1 moussante suivante par simple dissolution sous agitation de la quantité adéquate de polymère à l'état de poudre dans une solution de NaCl à 0,2 M dans l'eau, à température ambiante, pour obtenir la concentration en masse voulue du polymère, soit 3 % en poids.

### Formule 1

Polymère 1 dans l'eau à 3 % (en poids), NaCl 0,2 M.

### Procédé d'obtention de la mousse

Les mousses ont été obtenues à partir de la solution aqueuse de polymère préparée ci-dessus (5 g de solution dans des pilluliers de 10 ml) soumise à une agitation à l'aide d'un appareil DIAX 600 (Heidolph) pendant 5 min. à 8000 RPM, puis pendant 1 min. à 13500 RPM. L'axe utilisé a un diamètre extérieur de 10 mm (référence 10F).

### Stabilité de la mousse obtenue

L'évolution de l'aspect macroscopique des mousses ainsi obtenues a été suivi dans le temps à t = 0, 1 h et 2 h 30 min. ; le pouvoir moussant est d'autant plus grand que la hauteur de mousse est importante.

A 15°C, la mousse occupe initialement 100 % du volume. Après 1 heure, 50 % du volume est sous forme de mousse. Après 2 h 30, il n'y a plus de mousse.

A 50°C, la mousse occupe initialement 100 % du volume. Après 1 heure, 95 % de mousse reste. Après 2 h 30, 90 % du volume est encore une mousse.

Le polymère 1 permet en solution aqueuse à une faible concentration (Cp = 3 %, NaCl = 0,2 _{M}) d'obtenir un système moussant, de 15 à 50°C, sans tensioactif. La stabilité de la mousse obtenue est meilleure à 50 qu'à 15°C, ce comportement pouvant être corrélé aux propriétés de gélification par chauffage du Polymère 1.

### Mesure de la viscosité

Les mesures rhéologiques ont été réalisées à l'aide d'un rhéomètre Haake RS150 équipé d'une géométrie cône/plan (35 mm, 2°) et d'un bain thermostaté afin de contrôler la température entre 5 et 80°C. Les mesures ont été effectuées dans le mode écoulement, à une vitesse de cisaillement imposée égale à 10 s⁻¹, en faisant varier la température de 15 à 50°C à la vitesse de 0,5°C/min..

La solution du Polymère 1 à 3% (en poids), NaCl 0,2 mole/l, présente les caractéristiques suivantes :
- viscosité à 15°C (1s⁻¹) = 0,01 Pa.s ;
- viscosité à 50°C (1s⁻¹) = 0,5 Pa.s.

Le pouvoir gélifiant du Polymère 1, au-delà de 25°C, permet d'obtenir une mousse gélifiée dont l'application sur la peau est plus facile que pour une formule fluide.

### Exemple 2

Cet exemple est relatif à des formules moussantes sans tensioactif contenant le polymère 2. On étudie les stabilités à 15 et 38°C des mousses obtenues à partir de ces formules, ainsi que leur viscosité à ces températures.

### Préparation de la formule moussante

On prépare la formule moussante 2 de la même manière que dans l'exemple 1, mais on utilise de l'eau pure et la concentration du polymère est de 1 % en poids.

### Formule 2

Polymère 2 dans l'eau à 1% (en poids).

### Procédé d'obtention de la mousse

Les mousses ont été obtenues à partir de la solution aqueuse de polymère préparée ci-dessus (5 g de solution dans des pilluliers de 10 ml) soumise à une agitation à l'aide d'un appareil DIAX 600 (Heidolph) pendant 5 min. à 8 000 RPM, puis pendant 1 min. à 13 500 RPM. L'axe utilisé a un diamètre extérieur de 10 mm (référence 10F).

### Stabilité de la mousse obtenue

L'évolution de l'aspect macroscopique des mousses ainsi obtenues a été suivi dans le temps à t = 30 h et 27 j pour T = 15°C et à t = 51 h et 27 j pour T = 38°C ; le pouvoir moussant est d'autant plus grand que la hauteur de mousse est importante.

A 15°C, l'ensemble de la composition est sous forme de mousse. Après 30 h, 50 % est une mousse et après 27 j, il n'y a plus de mousse.

A 38°C, 100 % de la composition est une mousse. Après 51 h, il y a toujours 100 % de mousse. Après 27 j, il reste 50 % de mousse.

Le polymère 2 permet en solution aqueuse à une faible concentration (Cp = 1 %) d'obtenir un système moussant, de 15 à 38°C, sans tensioactif. La stabilité de la mousse obtenue est meilleure à 38 qu'à 15°C, ce comportement pouvant être corrélé aux propriétés de gélification par chauffage du Polymère 2.

### Mesure de la viscosité

Les mesures rhéologiques ont été réalisées à l'aide d'un rhéomètre Haake RS150 équipé d'une géométrie cône/plan (35 mm, 2°) et d'un bain thermostaté afin de contrôler la température entre 5 et 80°C. Les mesures ont été effectuées dans le mode écoulement, à une vitesse de cisaillement imposée égale à 10 s⁻¹, en faisant varier la température de 20 à 40°C à la vitesse de 0,5°C/min..

La figure suivante (figure 1) présente l'évolution de la viscosité de 20 à 40°C d'une solution aqueuse du Polymère 2 à 1 % (en poids).

Effet de thermogélification au-delà de 27°C :
- à 25°C : 0,15 Pa.s.
- à 38°C : 0,78 Pa.s.

Le pouvoir gélifiant du Polymère 2, au-delà de 27°C, permet d'obtenir une mousse gélifiée dont l'application sur la peau est plus facile que pour une formule fluide.

### Exemple 3

Cet exemple est relatif à des formules moussantes contenant 3 % d'un tensioactif alkyl polyglucoside et 1 % du polymère 2.

On étudie les stabilités à 15 et 38°C des mousses obtenues à partir de ces formules ainsi que leur viscosité à ces températures.

Le tensioactif utilisé est un alkyl C₁₀₋₁₄ polyglucoside commercialisé sous le nom de Oramix NS10 par Seppic.

Préparation de la formule moussante : on prépare les formules moussantes suivantes de la même manière que dans l'exemple 1.

### Formule 3A

Tensioactif à 3 % (en poids) + polymère 2 à 1 % en solution aqueuse.

### Formule 3B

Tensioactif à 3 % (en poids) en solution aqueuse.

### Procédé d'obtention de la mousse

Les mousses ont été obtenues à partir de solutions aqueuses de tensioactif en présence ou non des polymères, préparées ci-dessus (5 g de solution dans des pilluliers de 10 ml) soumises à une agitation à l'aide d'un appareil DIAX 600 (Heidolph) pendant 5 min. à 8 000 RPM, puis pendant 1 min. à 13500 RPM. L'axe utilisé a un diamètre extérieur de 10 mm (référence 10F) .

### Stabilité des mousses obtenues

L'évolution de l'aspect macroscopique des mousses ainsi obtenues a été suivi dans le temps à t = 0 et t = 7 h à 15 et 38°C ; le pouvoir moussant est d'autant plus grand que la hauteur de mousse est importante.

A 15°C, 100 % des compositions 3A et 3B sont sous forme de mousse. Après 7 h, il reste 70 % de mousse dans les deux cas.

A 38°C, 100 % des compositions 3A et 3B sont sous forme de mousse. Après 7 h, il reste 100 % de mousse pour la formule 3A et seulement 50 % pour la formule 3B.

L'introduction du Polymère 2 à une faible concentration (Cp = 1 %) dans une formule moussante contenant 3 % de tensioactif alkyl polyglucoside permet d'améliorer la stabilité de la mousse au-delà de la température de gélification du Polymère 2 (27°C pour une concentration dans l'eau égale à 1 %). La stabilité de la mousse est meilleure à 38 qu'à 15°C, ce comportement pouvant être corrélé aux propriétés de gélification par chauffage du Polymère 2.

### Mesures de la viscosité

Les mesures rhéologiques ont été réalisées à l'aide d'un rhéomètre Haake RS150 équipé d'une géométrie cône/plan (35 mm, 2°) et d'un bain thermostaté afin de contrôler la température entre 5 et 80°C. Les mesures ont été effectuées dans le mode écoulement, à une vitesse de cisaillement imposée égale à 10 s⁻¹, en faisant varier la température de 20 à 40°C à la vitesse de 0,5°C/min..

La figure suivante (figure 2) présente l'évolution de la viscosité de 20 à 45°C d'une solution aqueuse contenant le tensioactif (3 % en poids) et le Polymère 2 (1 % en poids).

Effet thermogélifiant à partir de 25°C :
- à 20°C : 0,13 Pa.s.
- à 38°C : 0,63 Pa.s.

### Exemple 4

Dans cet exemple, on prépare une composition cosmétique moussante fluide, qui gélifie à l'application.

Cette composition est la suivante (% en poids).

| | |
|---|---|
| Glycérine | 5 % |
| Polymère 1 | 3 % |
| C₁₀₋₁₄ polyglucoside (Oramix NS 10 de Seppic) | 5 % |
| Conservateur | 0,4 % |
| Ethylène diamine tétraacétate de sodium | 0,2 % |
| Eau déminéralisée | 86,4 % |

### Mode de préparation

Cette composition moussante est obtenue par dissolution du polymère 1 sous forme de poudre dans l'eau déminéralisée sous agitation à la température ambiante pendant 3 h ; les autres constituants sont alors introduits dans cette solution et l'agitation est maintenue pendant 30 min..

La formule obtenue est une composition moussante fluide à la température ambiante et qui gélifie à l'application sur la peau ; ce changement de texture est agréable et facilite l'application.

### REFERENCES

[1] D. HOURDET et al., Polymer, 1994, vol. 35, n° 12, pages 2 624-2 630.
[2] F. L'ALLORET et al., Coll. Polym. Sci., 1995, vol. 273, n° 12, pages 1 163-1 173.
[3] F. L'ALLORET, Revue de l'Institut Français du Pétrole, 1997, vol. 52, n° 2, pages 117-128.
[4] EP-A-0 583 814.
[5] EP-A-0 629 649.
[6] WO-A-95 24430.
[7] US-A-5 939 485.
[8] WO-A-97 00275.
[9] WO-A-98 48768.
[10] WO-A-00 35961.
[11] Articles de TAYLOR et al., Journal of Polymer Science, part A : Polymer Chemistry, 1975, 13, 2 551.
[12] J. BAILEY et al., Journal of Applied Polymer Science, 1959, 1,56.
[13] HESKINS et al., Journal of Macromolecular Science, Chemistry A2, 1968, 1 441.
[14] EP-A-0 566 438.
[15] FR-A-2 739 556.

## Revendications

1. Composition moussante thermogélifiante comprenant une phase aqueuse, ladite phase aqueuse comprenant un polymère comprenant des unités hydrosolubles et des unités présentant dans l'eau une température inférieure critique de démixtion LCST, la température de démixtion par chauffage en solution aqueuse desdites unités à LCST étant de 5 à 40°C pour une concentration massique dans l'eau de 1 % desdites unités, et la concentration dudit polymère dans ladite composition étant telle que sa température de gélification soit dans la plage de 5 à 40°C.

2. Composition selon la revendication 1, dans laquelle la température de démixtion par chauffage en solution aqueuse des unités à LCST du polymère est de 10 à 35°C pour une concentration massique dans l'eau de 1 % desdites unités.

3. Composition selon la revendication 2, dans laquelle la concentration du polymère dans la composition est telle que sa température de gélification soit dans la plage de 10 à 35°C.

4. Composition selon la revendication 1, dans laquelle le polymère se présente sous la forme d'un polymère séquencé (ou blocs) comprenant des séquences constituées d'unités hydrosolubles alternées avec des séquences constituées d'unités à LCST, ou sous la forme d'un polymère greffé dont le squelette est formé d'unités hydrosolubles ledit squelette étant porteur de greffons constitués d'unités à LCST, lesdits polymères pouvant être partiellement réticulés.

5. Composition selon l'une quelconque des revendications précédentes, dans laquelle les unités hydrosolubles sont, en totalité ou en partie, susceptibles d'être obtenues par polymérisation, ou par polycondensation ou encore sont constituées, en totalité ou en partie, par des polymères naturels ou naturels modifiés.

6. Composition moussante selon la revendication 5, dans laquelle les unités hydrosolubles sont, en totalité ou en partie, susceptibles d'être obtenues par polymérisation, notamment radicalaire, d'au moins un monomère choisi parmi les monomères suivants :
- l'acide (méth)acrylique ;
- les monomères vinyliques de formule (I) suivante : 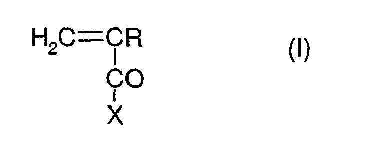
dans laquelle :
- R est choisi parmi H, -CH₃, -C₂H₅ ou -C₃H₇, et
- X est choisi parmi :
- les oxydes d'alkyle de type -OR' où R' est un radical hydrocarboné, linéaire ou ramifié, saturé ou insaturé, ayant de 1 à 6 atomes de carbone, éventuellement substitué par au moins un atome d'halogène (iode, brome, chlore, fluor); un groupement sulfonique (-SO₃⁻) , sulfate (-SO₄⁻) , phosphate (-PO₄H₂) ; hydroxy (-OH) ; amine primaire (-NH₂) ; amine secondaire (-NHR₁), tertiaire (-NR₁R₂) ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R' + R₁ + R₂ + R₃ ne dépasse pas 7 ; et
- les groupements -NH₂, -NHR₄ et -NR₄R₅ dans lesquels R₄ et R₅ sont indépendamment l'un de l'autre des radicaux hydrocarbonés, linéaires ou ramifiés, saturés ou insaturés ayant 1 à 6 atomes de carbone, sous réserve que le nombre total d'atomes de carbone de R₄ + R₅ ne dépasse pas 7, lesdits R₄ et R₅ étant éventuellement substitués par un atome d'halogène (iode, brome, chlore, fluor) ; un groupement hydroxy (-OH) ; sulfonique (-SO₃⁻) ; sulfate (-SO₄⁻) ; phosphate (-PO₄H₂) ; amine primaire (-NH₂) amine secondaire (NHR₁)' tertiaire (-NR₁R₂) et/ou quaternaire (-N⁺R₁R₂R₃) avec R₁, R₂ et R₃ étant, indépendamment l'un de l'autre, un radical hydrocarboné, linéaire ou ramifié, saturé ou insaturé ayant 1 à 6 atomes de carbone, sous réserve que la somme des atomes de carbone de R₄ + R₅ + R₁ + R₂ + R₃ ne dépasse pas 7 ;
- l'anhydride maléique ;
- l'acide itaconique ;
- l'alcool vinylique de formule CH₂=CHOH ;
- l'acétate de vinyle de formule CH₂=CH-OCOCH₃ ;
- les N-vinyllactames tels que la N-vinylpyrrolidone, la N-vinylcaprolactame et la N-butyrolactame ;
- les vinyléthers de formule CH₂=CHOR₆ dans laquelle R₆ est un radical hydrocarboné, linéaire ou ramifié, saturé ou insaturé, ayant de 1 à 6 atomes de carbone ;
- les dérivés hydrosolubles du styrène, notamment le styrène sulfonate ;
- le chlorure de diméthyldiallyl ammonium ; et
- la vinylacétamide.

7. Composition moussante selon la revendication 5, dans laquelle les unités hydrosolubles du polymère sont constituées en totalité ou en partie par des polycondensats ou par des polymères naturels ou naturels modifiés choisis parmi un ou plusieurs des composants suivants :
- les polyuréthannes hydrosolubles ;
- la gomme de xanthane ;
- les alginates et leurs dérivés tels que l'alginate de propylèneglycol ;
- les dérivés de cellulose et notamment la carboxyméthylcellulose, l'hydroxypropylcellulose, l'hydroxyéthylcellulose et l'hydroxyéthylcellulose quaternisée ;
- les galactomannanes et leurs dérivés tels que la gomme Konjac, la gomme de guar, l'hydroxypropylguar, l'hydroxypropylguar modifié par des groupements méthylcarboxylate de sodium, et le chlorure de guar hydroxypropyl triméthyl ammonium ; et
- la polyéthylène-imine.

8. Composition moussante selon l'une quelconque des revendications 5 à 7, dans laquelle les unités hydrosolubles du polymère ont une masse molaire allant de 1 000 g/mole à 5 000 000 g/mole lorsqu'elles constituent le squelette hydrosoluble d'un polymère greffé, ou une masse molaire allant de 500 g/mole à 100 000 g/mole lorsqu'elles constituent un bloc d'un polymère multiblocs.

9. Composition moussante selon l'une quelconque des revendications 1 à 8, dans laquelle les unités à LCST du polymère sont constituées d'un ou plusieurs polymères choisis parmi les polymères suivants :
- les polyéthers tels que le polyoxyde d'éthylène (POE), le polyoxyde de propylène (POP), et les copolymères statistiques d'oxyde d'éthylène (OE) et d'oxyde de propylène (OP) ;
- les polyvinylméthyléthers ;
- les dérivés polymériques et copolymériques N-substitués de l'acrylamide ayant une LCST ; et
- le polyvinylcaprolactame et les copolymères de vinyl caprolactame.

10. Composition moussante selon l'une quelconque des revendications 1 à 9, dans laquelle les unités à LCST du polymère sont constituées de poly oxyde de propylène (POP)ₙ où n est un nombre entier de 10 à 50, ou de copolymères statistiques d'oxyde d'éthylène (OE) et d'oxyde de propylène (OP), représentés par la formule :
(OE)ₘ (OP)ₙ
dans laquelle m est un nombre entier allant de 1 à 40, de préférence de 2 à 20, et n est un nombre entier allant de 10 à 60, de préférence de 20 à 50.

11. Composition moussante selon la revendication 10, dans laquelle la masse molaire des unités à LCST du polymère est de 500 à 5 300 g/mole, de préférence de 1 500 à 4 000 g/mole.

12. Composition moussante selon l'une quelconque des revendications 1 à 9, dans laquelle les unités à LCST du polymère sont constituées par un polymère choisi parmi le poly N-isopropylacrylamide, le poly N-éthyl acrylamide et les copolymères de N-isopropylacrylamide ou de N-éthylacrylamide et d'un monomère vinylique choisi parmi les monomères ayant la formule (I) donnée dans la revendication 6, l'anhydride maléique, l'acide itaconique, la vinylpyrrolidone, le styrène et ses dérivés, le chlorure de diméthyldiallyl ammonium, la vinylacétamide, l'alcool vinylique, l'acétate de vinyle, les vinyléthers et les dérivés de l'acétate de vinyle.

13. Composition moussante selon la revendication 12, dans laquelle la masse molaire des unités à LCST du polymère est de 1 000 g/mole à 500 000 g/mole, de préférence de 2 000 à 50 000 g/mole.

14. Composition moussante selon l'une quelconque des revendications 1 à 9, dans lequel les unités à LCST du polymère sont constituées par un polyvinylcaprolactame ou un copolymère de vinylcaprolactame et d'un monomère vinylique choisi parmi les monomères répondant à la formule (I) donnée dans la revendication 6, l'anhydride maléique, l'acide itaconique, la vinylpyrrolidone, le styrène et ses dérivés, le chlorure de diméthyldiallyl ammonium, la vinylacétamide, l'alcool vinylique, l'acétate de vinyle, les vinyléthers et les dérivés de l'acétate de vinyle.

15. Composition moussante selon la revendication 14, dans lequel la masse molaire des unités à LCST est de 1 000 à 500 000 g/mole, de préférence de 2 000 à 50 000 g/mole.

16. Composition moussante selon l'une quelconque des revendications 1 à 15, dans laquelle la proportion massique des unités à LCST du polymère est de 5 à 70 %, de préférence de 20 à 65 % et mieux encore de 30 à 60 %, par rapport au polymère.

17. Composition moussante selon l'une quelconque des revendications 1 à 16, dans laquelle la concentration massique en polymère de la phase aqueuse est de 0,1 à 20 %.

18. Composition moussante selon l'une quelconque des revendications 1 à 17, dans laquelle la phase aqueuse comprend, en outre, éventuellement un tensioactif moussant.

19. Composition moussante selon la revendication 18, dans laquelle ledit tensioactif moussant est non ionique.

20. Composition selon l'une quelconque des revendications 1 à 19, dans laquelle la phase aqueuse est constituée par un milieu physiologiquement acceptable permettant une application topique et notamment cosmétique.

21. Composition selon la revendication précédente, **caractérisée en ce qu'**elle constitue un gel douche, un nettoyant pour le visage, un produit démaquillant, un shampooing, une mousse ou un gel à raser.

22. Mousse susceptible d'être obtenue à partir de la composition moussante selon l'une quelconque des revendications 1 à 21, formée d'une dispersion de bulles de gaz dans la phase aqueuse continue.

23. Utilisation du polymère tel que décrit dans la revendication 1, pour stabiliser une mousse à une température supérieure à sa température de gélification.

24. Utilisation cosmétique d'une composition selon l'une quelconque des revendications 1 à 21, pour le nettoyage et/ou le démaquillage de la peau, du cuir chevelu, des ongles, des cils, des sourcils, des yeux, des muqueuses, des semi-muqueuses et/ou des cheveux.

25. Procédé cosmétique de nettoyage et/ou de démaquillage des matières kératiniques (peau, cuir chevelu, ongles, cils, sourcils, yeux, muqueuses, semi-muqueuses et/ou cheveux), **caractérisé par le fait qu'**on applique la composition selon l'une quelconque des revendications 1 à 21, sur les matières kératiniques, en présence d'eau, et qu'on élimine la mousse formée et les résidus de salissure par rinçage à l'eau.

## Claims

1. Heat-induced gelling foaming composition comprising an aqueous phase, said aqueous phase comprising a polymer comprising water-soluble units and units having in water a lower critical solution temperature LCST, the heat-induced demixing temperature in aqueous solution of said units with an LCST being from 5 to 40°C for a concentration of said units in water of 1% by mass, and the concentration of said polymer in said composition being such that its gel point is in the range from 5 to 40°C.

2. Composition according to Claim 1, in which the heat-induced demixing temperature in aqueous solution of the units with an LCST of the polymer is from 10 to 35°C for a concentration by mass in water of 1% of the said units.

3. Composition according to Claim 2, in which the concentration of the polymer in the composition is such that its gel point is in the range from 10 to 35°C.

4. Composition according to Claim 1, in which the polymer is in the form of a block polymer comprising blocks consisting of water-soluble units alternating with blocks consisting of units with an LCST, or in the form of a grafted polymer whose backbone is formed by water-soluble units, said backbone bearing grafts consisting of units with an LCST, said polymers possibly being partially crosslinked.

5. Composition according to any one of the preceding claims, in which the water-soluble units are totally or partially capable of being obtained by polymerization, or by polycondensation, or alternatively consist totally or partially of natural polymers or modified natural polymers.

6. Foaming composition according to Claim 5, in which the water-soluble units are totally or partially capable of being obtained by polymerization, especially free-radical polymerization, of at least one monomer chosen from the following monomers:
- (meth)acrylic acid;
- vinyl monomers of formula (I) below: 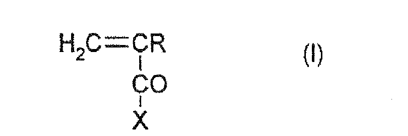
in which:
- R is chosen from H, -CH₃, -C₂H₅ Or -C₃H₇, and
- X is chosen from:
- alkyl oxides of -OR' type in which R' is a linear or branched, saturated or unsaturated hydrocarbon-based radical containing from 1 to 6 carbon atoms, optionally substituted with at least one halogen atom (iodine, bromine, chlorine or fluorine); a sulphonic (-SO₃⁻), sulphate (-SO₄⁻), phosphate (-PO₄H₂); hydroxyl (-OH); primary amine (-NH₂); secondary amine (-NHR₁), tertiary amine (-NR₁R₂) or quaternary amine (-N⁺R₁R₂R₃) group with R₁, R₂ and R₃ being, independently of each other, a linear or branched, saturated or unsaturated hydrocarbon-based radical containing 1 to 6 carbon atoms, with the proviso that the sum of the carbon atoms of R' + R₁ + R₂ + R₃ does not exceed 7; and
- -NH₂, -NHR₄ and -NR₄R₅ groups in which R₄ and R₅ are, independently of each other, linear or branched, saturated or unsaturated hydrocarbon-based radicals containing 1 to 6 carbon atoms, with the proviso that the total number of carbon atoms of R₄ + R₅ does not exceed 7, the said R₄ and R₅ optionally being substituted with a halogen atom (iodine, bromine, chlorine or fluorine); a hydroxyl (-OH); sulphonic (-SO₃⁻); sulphate (-SO₄⁻); phosphate (-PO₄H₂); primary amine (-NH₂); secondary amine (-NHR₁), tertiary amine (-NR₁R₂) and/or quaternary amine (-N⁺R₁R₂R₃) group with R₁, R₂ and R₃ being, independently of each other, a linear or branched, saturated or unsaturated hydrocarbon radical containing 1 to 6 carbon atoms, with the proviso that the sum of the carbon atoms of R₄ + R₅ + R₁ + R₂ + R₃ does not exceed 7;
- maleic anhydride;
- itaconic acid;
- vinyl alcohol of formula CH₂=CHOH;
- vinyl acetate of formula CH₂=CH-OCOCH₃;
- N-vinyllactams such as N-vinylpyrrolidone, N-vinylcaprolactam and N-butyrolactam;
- vinyl ethers of formula CH₂=CHOR₆ in which R₆ is a linear or branched, saturated or unsaturated hydrocarbon radical containing from 1 to 6 carbon atoms;
- water-soluble styrene derivatives, especially styrene sulphonate;
- dimethyldiallylammonium chloride; and
- vinylacetamide.

7. Foaming composition according to Claim 5, in which the water-soluble units of the polymer consist totally or partially of polycondensates or of natural polymers or modified natural polymers chosen from one or more of the following components:
- water-soluble polyurethanes;
- xanthan gum;
- alginates and derivatives thereof such as propylene glycol alginate;
- cellulose derivatives and especially carboxymethylcellulose, hydroxypropylcellulose, hydroxyethylcellulose and quaternized hydroxyethylcellulose;
- galactomannans and derivatives thereof, such as Konjac gum, guar gum, hydroxypropylguar, hydroxypropylguar modified with sodium methylcarboxylate groups, and hydroxypropyltrimethylammonium guar chloride; and
- polyethyleneimine.

8. Foaming composition according to any one of Claims 5 to 7, in which the water-soluble units of the polymer have a molar mass ranging from 1000 g/mol to 5 000 000 g/mol when they constitute the water-soluble backbone of a grafted polymer, or a molar mass ranging from 500 g/mol to 100 000 g/mol when they constitute a block of a multiblock polymer.

9. Foaming composition according to any one of Claims 1 to 8, in which the units with an LCST of the polymer consist of one or more polymers chosen from the following polymers:
- polyethers such as polyethylene oxide (PEO), polypropylene oxide (PPO) or random copolymers of ethylene oxide (EO) and of propylene oxide (PO) ;
- polyvinyl methyl ethers ;
- polymeric and copolymeric N-substituted acrylamide derivatives with an LCST; and
- polyvinylcaprolactam and vinylcaprolactam copolymers.

10. Foaming composition according to any one of Claims 1 to 9, in which the units with an LCST of the polymer consist of polypropylene oxide (PPO)ₙ with n being an integer from 10 to 50, or of random copolymers of ethylene oxide (EO) and of propylene oxide (PO), represented by the formula:
(EO)ₘ(PO)ₙ
in which m is an integer ranging from 1 to 40 and preferably from 2 to 20, and n is an integer ranging from 10 to 60 and preferably from 20 to 50.

11. Foaming composition according to Claim 10, in which the molar mass of the units with an LCST of the polymer is from 500 to 5300 g/mol and preferably from 1 500 to 4000 g/mol.

12. Foaming composition according to any one of Claims 1 to 9, in which the units with an LCST of the polymer consist of a polymer chosen from poly-N-isopropylacrylamide, poly-N-ethylacrylamide and copolymers of N-isopropylacrylamide or of N-ethylacrylamide and of a vinyl monomer chosen from monomers having the formula (I) given in Claim 6, maleic anhydride, itaconic acid, vinylpyrrolidone, styrene and its derivatives, dimethyldiallylammonium chloride, vinylacetamide, vinyl alcohol/vinyl acetate, vinyl ethers and vinyl acetate derivatives.

13. Foaming composition according to Claim 12, in which the molar mass of the units with an LCST of the polymer is from 1000 g/mol to 500 000 g/mol and preferably from 2000 to 50 000 g/mol.

14. Foaming composition according to any one of Claims 1 to 9, in which the units with an LCST of the polymer consist of a polyvinylcaprolactam or a copolymer of vinylcaprolactam and of a vinyl monomer chosen from the monomers corresponding to formula (I) given in Claim 6, maleic anhydride, itaconic acid, vinylpyrrolidone, styrene and its derivatives, dimethyldiallylammonium chloride, vinylacetamide, vinyl alcohol, vinyl acetate, vinyl ethers and vinyl acetate derivatives.

15. Foaming composition according to Claim 14, in which the molar mass of the units with an LCST is from 1000 to 500 000 g/mol and preferably from 2000 to 50 000 g/mol.

16. Foaming composition according to any one of Claims 1 to 15, in which the proportion by mass of units with an LCST of the polymer is from 5 to 70%, preferably from 20 to 65% and better still from 30 to 60% relative to the polymer.

17. Foaming composition according to any one of Claims 1 to 16, in which the concentration by mass of polymer in the aqueous phase is from 0.1 to 20%.

18. Foaming composition according to any one of Claims 1 to 17, in which the aqueous phase also optionally comprises a foaming surfactant.

19. Foaming composition according to Claim 18, in which said foaming surfactant is nonionic.

20. Composition according to any one of Claims 1 to 19, in which the aqueous phase consists of a physiologically acceptable medium allowing a topical application and especially a cosmetic application.

21. Composition according to the preceding claim, **characterized in that** it constitutes a shower gel, a facial cleansing product, a make-up-removing product, a shampoo or a shaving foam or gel.

22. Foam which may be obtained from the foaming composition according to any one of Claims 1 to 21, formed from a dispersion of gas bubbles in the continuous aqueous phase.

23. Use of the polymer as described in Claim 1 to stabilize a foam at a temperature above its gel point.

24. Cosmetic use of a composition according to any one of Claims 1 to 21, for cleansing and/or removing make-up from the skin, the scalp, the nails, the eyelashes, the eyebrows, the eyes, mucous membranes, semi-mucous membranes and/or the hair.

25. Cosmetic process for cleansing and/or removing make-up from keratin materials (skin, scalp, nails, eyelashes, eyebrows, eyes, mucous membranes, semi-mucous membranes and/or hair), **characterized in that** the composition according to any one of Claims 1 to 21 is applied to the keratin materials, in the presence of water, and the foam formed and the soiling residues are removed by rinsing with water.

## Patentansprüche

1. Thermogelierende Schaumzusammensetzung, enthaltend eine wässrige Phase, wobei die wässrige Phase ein Polymeres enthält, welches wasserlösliche Einheiten und Einheiten, die in Wasser eine untere kritische Entmischungs- (Lösungs-) temperatur LCST aufweisen, enthält, wobei die Entmischungstemperatur bei Erwärmen der LCST-Einheiten in wässriger Lösung bei einer Massenkonzentration der Einheiten in Wasser von 1 % dieser Einheiten 5 bis 40 °C beträgt, und die Konzentration des Polymeren in der Zusammensetzung derart ist, dass seine Gelierungstemperatur in dem Bereich von 5 bis 40 °C liegt.

2. Zusammensetzung gemäß Anspruch 1, wobei die Entmischungstemperatur bei Erwärmen der LCST-Einheiten des Polymeren in wässriger Lösung bei einer Massenkonzentration der Einheiten in Wasser von 1 % 10 bis 35 °C beträgt.

3. Zusammensetzung gemäß Anspruch 2, wobei die Konzentration des Polymeren in der Zusammensetzung derart ist, dass seine Gelierungstemperatur im Bereich von 10 bis 35 °C liegt.

4. Zusammensetzung gemäß Anspruch 1, wobei das Polymere in Form eines Sequenz- (oder Block-) Polymeren vorliegt, das Sequenzen enthält, die aus wasserlöslichen Einheiten alternierend mit Sequenzen aus LCST-Einheiten bestehen, oder in Form eines Pfropfpolymeren vorliegt, dessen Gerüst aus wasserlöslichen Einheiten gebildet wird, wobei das Gerüst Pfropfe trägt, die aus LCST-Einheiten bestehen, wobei die Polymeren teilweise vernetzt sein können.

5. Zusammensetzung gemäß wenigstens einem der vorherigen Ansprüche, wobei die wasserlöslichen Einheiten, insgesamt oder teilweise, durch Polymerisation oder durch Polykondensation erhältlich sind, oder, insgesamt oder teilweise, aus natürlichen oder modifizierten natürlichen Polymeren bestehen.

6. Schaumzusammensetzung gemäß Anspruch 5, wobei die wasserlöslichen Einheiten, insgesamt oder teilweise, durch Polymerisation, insbesondere radikalische Polymerisation, wenigstens eines Monomeren, ausgewählt aus den folgenden Monomeren, erhältlich sind:
- (Meth)acrylsäure,
- Vinylmonomere mit folgender Formel (1): 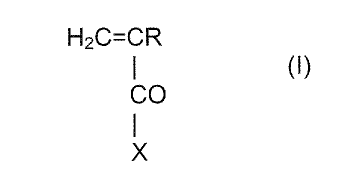
wobei:
- R ausgewählt ist aus H, -CH₃, -C₂H₅ oder -C₃H₇, und
- X ausgewählt ist aus:
- Alkyloxiden des Typs -OR', wobei R' ein linearer oder verzweigter, gesättigter oder ungesättigter Kohlenwasserstoffrest mit 1 bis 6 Kohlenstoffatomen ist, der gegebenenfalls mit wenigstens einem Halogenatom (lod, Brom, Chlor, Fluor), einer Sulfongruppe (-SO₃-), Sulfatgruppe (-SO₄-), Phosphatgruppe (-PO₄H₂), Hydroxygruppe (-OH), einem primären Amin (-NH₂), sekundären Amin (-NHR₁), tertiären Amin (-NR₁R₂) oder quaternären Amin (-N⁺R₁R₂R₃) substituiert ist, wobei R₁, R₂ und R₃ unabhängig voneinander lineare oder verzweigte, gesättigte oder ungesättigte Kohlenwasserstoffreste mit 1 bis 6 Kohlenstoffatomen sind, unter der Voraussetzung, dass die Summe der Kohlenstoffatome von R' + R₁ + R₂ + R₃ 7 nicht übersteigt, und
- den Gruppen -NH₂, -NHR₄ und -NR₄R₅, wobei R₄ und R₅ unabhängig voneinander lineare oder verzweigte, gesättigte oder ungesättigte Kohlenwasserstoffreste mit 1 bis 6 Kohlenstoffatomen sind, unter der Voraussetzung, dass die Summe der Kohlenstoffatome von R₄ + R₅ 7 nicht übersteigt, wobei R₄ und R₅ gegebenenfalls mit einem Halogenatom (lod, Brom, Chlor, Fluor), einer Hydroxygruppe (-OH), Sulfongruppe (-SO₃-), Sulfatgruppe (-SO₄⁻), Phosphatgruppe (-PO₄H₂), einem primären Amin (-NH₂), sekundären Amin (-NHR₁), tertiären Amin (-NR₁R₂) und/oder quaternären Amin (-N⁺R₁R₂R₃) substituiert sind, wobei R₁, R₂ und R₃ unabhängig voneinander lineare oder verzweigte, gesättigte oder ungesättigte Kohlenwasserstoffreste mit 1 bis 6 Kohlenstoffatomen sind, unter der Voraussetzung, dass die Summe der Kohlenstoffatome von R₄ + R₅ + R₁ + R₂ + R₃ 7 nicht übersteigt,
- Maleinsäureanhydrid,
- Itakonsäure,
- Vinylalkohol der Formel CH₂ =CHOH,
- Vinylacetat der Formel CH₂ =CH-OCOCH₃
- N-Vinyllactame, wie beispielsweise, N-Vinylpyrrolidon, N-Vinylcaprolactam und N-Butyrolactam,
- Vinylether der Formel CH₂=CHOR₆, worin R₆ ein linearer oder verzweigter, gesättigter oder ungesättigter Kohlenwasserstoffrest mit 1 bis 6 Kohlenstoffatomen ist,
- wasserlösliche Styrol-Derivate, insbesondere Styrolsulfonat,
- Dimethyldiallyl-Ammoniumchlorid und
- Vinylacetamid.

7. Schaumzusammensetzung gemäß Anspruch 5, wobei die wasserlöslichen Einheiten des Polymeren insgesamt oder teilweise aus Polykondensaten oder aus natürlichen oder modifizierten natürlichen Polymeren bestehen, die aus einem oder mehreren der folgenden Komponenten ausgewählt sind:
- wasserlöslichen Polyurethanen,
- Xanthangummi,
- Alginaten und ihren Derivaten, wie beispielsweise Propylenglykol-Alginat,
- Zellulose-Derivaten, insbesondere Carboxymethylzellulose, Hydroxypropylzellulose, Hydroxyethylzellulose und quaternisierter Hydroxyethylzellulose,
- Galaktomannanen und ihren Derivaten, wie beispielsweise Konjac-Gummi, Guargummi, Hydroxypropylguar, mit Natriummethylcarboxylat-Gruppen modifiziertem Hydroxypropylguar und Guar-Hydroxypropyl-Trimethyl-Ammoniumchlorid, und
- Polyethylen-Imin.

8. Schaumzusammensetzung gemäß wenigstens einem der Ansprüche 5 bis 7, wobei die wasserlöslichen Einheiten des Polymeren eine molare Masse von 1.000 g/mol bis 5.000.000 g/mol, wenn sie das wasserlösliche Gerüst eines Pfropfpolymeren bilden, oder eine molare Masse von 500 g/mol bis 100.000 g/mol, wenn sie einen Block eines Multiblock-Polymeren bilden, aufweisen.

9. Schaumzusammensetzung gemäß wenigstens einem der Ansprüche 1 bis 8, wobei die LCST-Einheiten des Polymeren aus einem oder mehreren der folgenden Polymeren ausgewählt sind:
- Polyethern, wie beispielsweise Polyethylenoxid (PEO), Polypropylenoxid (PPO) und Zufalls-Copolymere von Ethylenoxid (EO) und Propylenoxid (PO),
- Polyvinylmethylethern,
- N-substituierten Polymer- und Copolymer-Derivaten von Acrylamid mit einer LCST und
- Polyvinylcaprolactam und Copolymeren von Vinylcaprolactam.

10. Schaumzusammensetzung gemäß wenigstens einem der Ansprüche 1 bis 9, wobei die LCST-Einheiten des Polymeren aus Polypropylenoxid (PPO)ₙ, wobei n eine ganze Zahl von 10 bis 50 ist, oder Zufalls-Copolymeren aus Ethylenoxid (EO) und Propylenoxid (PO) gemäß der Formel:
(EO)ₘ (PO)ₙ
wobei m eine ganze Zahl von 1 bis 40, bevorzugt von 2 bis 20, ist, und n eine ganze Zahl von 10 bis 60, bevorzugt von 20 bis 50, ist, bestehen.

11. Schaumzusammensetzung gemäß Anspruch 10, wobei die molare Masse der LCST-Einheiten des Polymeren 500 bis 5.300 g/mol, bevorzugt 1.500 bis 4.000 g/mol, beträgt.

12. Schaumzusammensetzung gemäß wenigstens einem der Ansprüche 1 bis 9, wobei die LCST-Einheiten des Polymeren aus einem Polymeren bestehen, das ausgewählt ist aus Poly-N-Isopropylacrylamid, Poly-N-Ethylacrylamid und den Copolymeren aus N-Ispropylacrylamid oder N-Ethylacrylamid und einem Vinylmonomeren, ausgewählt aus den Monomeren gemäß Formel (I) in Anspruch 6, Maleinsäureanhydrid, Itakonsäure, Vinylpyrrolidon, Styrol und seinen Derivaten, Dimethyldiallyl-Ammoniumchlorid, Vinylacetamid, Vinylalkohol, Vinylacetat, Vinylethern und Vinylacetat-Derivaten.

13. Schaumzusamensetzung gemäß Anspruch 12, wobei die molare Masse der LCST-Einheiten des Polymeren 1.000 bis 500.000 g/mol, bevorzugt von 2.000 bis 50.000 g/mol, beträgt.

14. Schaumzusammensetzung gemäß wenigstens einem der Ansprüche 1 bis 9, wobei die LCST-Einheiten des Polymeren aus einem Polyvinylcaprolactam oder einem Copolymeren aus Vinylcaprolactam und einem Vinylmonomeren, ausgewählt aus den Monomeren gemäß Formel (I) in Anspruch 6, Maleinsäureanhydrid, ltakonsäure, Vinylpyrrolidon, Styrol und seinen Derivaten, Dimethyldiallyl-Ammoniumchlorid, Vinylacetamid, Vinylalkohol, Vinylacetat, Vinylethern und Vinylacetat-Derivaten, bestehen.

15. Schaumzusammensetzung gemäß Anspruch 14, wobei die molare Masse der LCST-Einheiten 1.000 bis 500.000 g/mol, bevorzugt 2.000 bis 50.000 g/mol, beträgt.

16. Schaumzusammensetzung gemäß wenigstens einem der Ansprüche 1 bis 15, wobei der Massenanteil der LCST-Einheiten des Polymeren 5 bis 70 %, bevorzugt 20 bis 65 %, noch bevorzugter 30 bis 60 %, bezogen auf das Polymere, beträgt.

17. Schaumzusammensetzung gemäß wenigstens einem der Ansprüche 1 bis 16, wobei die Massenkonzentration des Polymeren der wässrigen Phase 0,1 bis 20 % beträgt.

18. Schaumzusammensetzung gemäß wenigstens einem der Ansprüche 1 bis 17, wobei die wässrige Phase außerdem gegebenenfalls ein grenzflächenaktives Schaummittel enthält.

19. Schaumzusammensetzung gemäß Anspruch 18, wobei das grenzflächenaktive Schaummittel nicht-ionisch ist.

20. Schaumzusammensetzung gemäß wenigstens einem der Ansprüche 1 bis 19, wobei die wässrige Phase aus einem physiologisch akzeptablen Milieu/Medium besteht, welches ein topisches, insbesondere kosmetisches, Auftragen ermöglicht.

21. Zusammensetzung gemäß dem vorherigen Anspruch, **dadurch gekennzeichnet**, das sie ein Duschgel, Gesichtsreinigungsmittel, Abschminkprodukt, Shampoo, einen Rasierschaum oder ein Rasiergel bildet.

22. Schaum, der ausgehend von der Schaumzusammensetzung gemäß wenigstens einem der Ansprüche 1 bis 21 erhältlich ist, gebildet aus einer Dispersion von Gasbläschen in der kontinuierlichen wässrigen Phase.

23. Verwendung des in Anspruch 1 beschriebenen Polymeren zur Stabilisierung eines Schaums bei einer Temperatur über seiner Gelierungstemperatur.

24. Kosmetische Verwendung einer Zusammensetzung gemäß wenigstens einem der Ansprüche 1 bis 21 zur Reinigung und/oder zum Abschminken der Haut, der Kopfhaut, der Nägel, der Wimpern, der Augenbrauen, der Augen, der Schleimhäute, der Halbschleimhäute und/oder der Haare.

25. Kosmetisches Verfahren zur Reinigung und/oder zum Abschminken von Keratin-Material (Haut, Kopfhaut, Nägel, Wimpern, Augenbrauen, Augen, Schleimhäute, Halbschleimhäute und/oder Haare), **dadurch gekennzeichnet, dass** die Zusammensetzung gemäß wenigstens einem der Ansprüche 1 bis 21 in Gegenwart von Wasser auf das Keratin-Material aufgetragen wird, und der gebildete Schaum und die Schmutzrückstände durch Spülen mit Wasser entfernt werden.
